# EUROPEAN PATENT SPECIFICATION

(11) **EP 3 937 662 B1**
(45) Date of publication and mention of the grant of the patent: **04.01.2023**
(21) Application number: 20810868.8
(22) Date of filing: 16.11.2020
(51) Int. Cl.: A24B 13/00, A61K 31/465

(54) **NON-TOBACCO ORAL NICOTINE POUCH COMPOSITION**
TABAKFREIE ORALE NIKOTINBEUTELZUSAMMENSETZUNG
COMPOSITION EN SACHET DE NICOTINE SANS TABAC DESTINÉE À ÊTRE ADMINISTRÉE PAR VOIE ORALE

(30) Priority: 05.06.2020 WO PCT/DK2020/050159; 05.06.2020 WO PCT/DK2020/050160; 05.06.2020 WO PCT/DK2020/050161; 05.06.2020 WO PCT/DK2020/050162; 05.06.2020 WO PCT/DK2020/050163
(43) Date of publication of application: 19.01.2022
(60) Divisional application: 21174121.0
(73) Proprietor: Philip Morris Products S.A., 2000 Neuchâtel (CH)
(72) Inventor: BRUUN, Heidi Ziegler, 7120 Veijle Øst (DK); JAKOBSEN, Bine Hare, 8680 Ry (DK); STAHL, My Ly Lao, 7120 Vejle Øst (DK)
(74) Representative: Taylor, Gillian Claire
(86) International application number: PCT/DK2020/050311
(87) International publication number: WO 2021/244714

(56) References cited:
- WO-A1-2012/134380
- WO-A1-2015/067372
- WO-A1-2019/115778

## Description

### FIELD OF INVENTION

The present invention relates to non-tobacco oral nicotine pouch compositions and oral pouched nicotine products according to the claims.

### BACKGROUND

Delivery of nicotine by smoking has many well-known drawbacks, in particular health related problems, such as inclusion of carcinogenic substances.

However, tobacco substitutes also suffer from disadvantages, such as inadequate relief of cravings for the user.

A further challenge in the prior art is that the desired release of nicotine should be attractive to the user of the pouch from a user perspective.

Yet at further challenge in relation to the prior art may be that pouches as delivery vehicle for nicotine may be somewhat costly and thereby impose restrictions on the way pouches are designed in order to keep manufacturing costs in check.

It is an object of one embodiment of the present invention to provide a nicotine containing pouch, e.g. as a tobacco substitute, which may solve the above problems.

Pouch compositions are known in the art.

One example is found in International Patent Application WO 2012/134380 A1, disclosing a product for oral delivery of nicotine comprising a core comprising a powder of at least one free nicotine salt, at least one pH adjusting agent and at least one filler.

Another example is found in International Patent application WO 2015/067372 A1, relating to oral smokeless tobacco products and oral smokeless non-tobacco snuff products comprising xylitol in an amount of from 6 to 20 w/w % of the final product.

Yet another example is found in International Patent Application WO 2019/115778 A1, relating to an oral pouched nicotine product comprising a moist filling material and a saliva-permeable pouch of a packaging material enclosing the moist filling material comprising comprises a particulate non-tobacco material, a non-encapsulated non-particulate flavouring agent, a nicotine source, a pH adjusting agent, a mono glyceride, and tobacco material.

### SUMMARY

The invention relates to a non-tobacco oral nicotine pouch composition comprising water in an amount of at least 15 % by weight of the pouch composition,
nicotine, and
at least one sugar alcohol,
wherein the pouch composition is free of humectants consisting of alginate, propylene glycol, hydroxypropyl cellulose and glycerol, and
wherein the at least one sugar alcohol comprises non-directly compressible (non-DC) grade sugar alcohol.

An advantage of the invention may be that a combination of a high nicotine release rate may be obtained together with a desirable mouthfeel, hereunder softness and moisture feeling.

A further advantage of the invention may be that a desirable flavor perception may be obtained, facilitated by a combination of an improved flavor release and a desirable mouthfeel, hereunder softness and moisture feeling.

Advantages of the present invention may be improved release of nicotine, improved release of flavor, and improved flavor sensation without compromising the moisture mouth feel.

An even further advantage of the invention is that the combination of sugar alcohol, and water provides not only an attractive mouthfeel but also a very attractive taste profile.

Traditionally, humectants may be added to protect products from drying out and may also have a preservative effect.

A high water content can make the products vulnerable to microbial growth, such as growth of yeast, mold, and bacteria. This may not only compromise health safety but also leads to significant deterioration of the user experience, by visually alteration of the product, altering and deterioration of the taste etc. Surprisingly, the inventors found that the pouch composition according to the invention had a desirable taste and mouthfeel, such as softness and moisture feeling, even after significant time of storage.

A further advantage of the invention may be a surprisingly effective retention of water. Normally, retention of water is provided by including a humectant such as alginate, propylene glycol, hydroxypropyl cellulose, or glycerol. However, even though the pouch composition of the invention is free of these substances as humectants, an advantageously effective retention of water may be obtained.

In an embodiment of the invention, the pouch composition is free of humectants in the form of sugar alcohols comprising no more than three carbons. Examples of sugar alcohols comprising no more than three carbons include glycerol, propylene glycol, and ethylene glycol.

In an advantageous embodiment of the invention, the pouch composition is further free of humectants consisting of modified starch, triacetin, polyethylene glycol (PEG), pectin, and xanthan gum.

Thus, in the above embodiment, the pouch composition is free of humectants consisting of alginate, propylene glycol, hydroxypropyl cellulose, glycerol, modified starch, triacetin, polyethylene glycol (PEG), pectin, and xanthan gum.

In an advantageous embodiment of the invention, the pouch composition comprises 0 - 0.4% by weight of humectants.

Thus, in the above embodiment, the pouch composition is free of humectants or comprises humectants in an amount of up to 0.4% by weight of the pouch composition.

In an advantageous embodiment of the invention, the pouch composition is free of humectants.

Thus, in the above embodiment, the pouch composition is free of any humectants, both alginate, sugar alcohols comprising no more than three carbons, hydroxypropyl cellulose, modified starch, triacetin, polyethylene glycol (PEG), pectin, xanthan gum and other humectants.

In an embodiment of the invention, the pouch composition comprises sugar alcohol in an amount of at least 1% by weight of the composition, such as at least 2% by weight of the composition, such as at least 5% by weight of the composition, such as at least 10% by weight of the composition, such as at least 15% by weight of the composition.

In an embodiment of the invention, the pouch composition comprises sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an advantageous embodiment of the invention, the pouch composition comprises the at least one sugar alcohol in an amount of at least 1% by weight of the composition, such as at least 2% by weight of the composition, such as at least 5% by weight of the composition, such as at least 10% by weight of the composition, such as at least 15% by weight of the composition.

In an advantageous embodiment of the invention, the pouch composition comprises the at least one sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the invention, the at least one sugar alcohol comprises sugar alcohol selected from the group consisting of xylitol, maltitol, mannitol, erythritol, isomalt, sorbitol, lactitol, hydrogenated starch hydrolysates, and any mixture thereof.

As an example embodiment, hydrogenated starch hydrolysates may be used, which comprises a mixture of primarily maltitol, sorbitol and further sugar alcohols.

According to the invention, the at least one sugar alcohol comprises non-directly compressible (non-DC) grade sugar alcohol.

Traditionally, direct compressible ingredients are used to increase flowability of a powdered composition. Typically, when handing powdered compositions, flowability is used as a measure of the processability. However, when adding significant amounts of water to a sugar alcohol containing composition, the composition tends to agglomerate and the flowability is drastically reduced, possibly even to the point where it cannot be measured by conventional methods. Surprisingly, the present inventors found that the pouch composition of the invention comprising non-DC sugar alcohol was still processable in filling machinery into individual pouches. Thus, the present invention facilitates effective processing and thereby a cost-effective setup by enabling the use of non-DC sugar alcohol and by avoiding e.g. filling of the pouches by hand or very complex, special designed filling machinery.

It is noted that different sugar alcohols may be applied for the purpose of taste and salivation, where the sugar alcohol composition is made of different sugar alcohols having different properties with respect to storage, processability and/or taste.

In an advantageous embodiment of the invention, the at least one non-directly compressible (non-DC) grade sugar alcohol is selected from the list consisting of xylitol, maltitol, mannitol, erythritol, isomalt, lactitol, and any combination thereof.

In an advantageous embodiment of the invention, the pouch composition comprises non-directly compressible (non-DC) grade sugar alcohol in an amount of at least 1% by weight of the composition, such as at least 2% by weight of the composition, such as at least 5% by weight of the composition, such as at least 10% by weight of the composition, such as at least 15% by weight of the composition.

In an advantageous embodiment of the invention, the pouch composition comprises non-directly compressible (non-DC) grade sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the pouch composition comprises non-directly compressible (non-DC) grade sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the pouch composition comprises non-directly compressible (non-DC) grade sugar alcohol in an amount of 1 to 80% by weight of the composition, such as 10 to 70% by weight of the composition, such as 10 to 60% by weight of the composition, such as 15 to 60% by weight of the composition, such as 20 to 60% by weight of the composition, such as 20 to 50% by weight of the composition.

In an embodiment of the invention, xylitol, maltitol, mannitol, erythritol, isomalt, lactitol, and mixtures thereof is used as the at least one non-directly compressible (non-DC) grade sugar alcohol.

In an embodiment of the invention, the at least one non-directly compressible (non-DC) grade sugar alcohol(s) are selected from the list consisting of xylitol, maltitol, mannitol, erythritol, isomalt, lactitol, and mixtures thereof.

In an embodiment of the invention, the pouch composition comprise at least one non-directly compressible (non-DC) grade sugar alcohol, such as at least two non-directly compressible (non-DC) grade sugar alcohols, such as at least three non-directly compressible (non-DC) grade sugar alcohols.

In an embodiment of the invention, the at least two non-directly compressible (non-DC) grade sugar alcohols are selected from the list consisting of xylitol, maltitol, mannitol, erythritol, isomalt, lactitol, and mixtures thereof.

In an embodiment of the invention, the at least three non-directly compressible (non-DC) grade sugar alcohols are selected from the list consisting of xylitol, maltitol, mannitol, erythritol, isomalt, lactitol, and mixtures thereof.

In an embodiment of the invention, the at least one sugar alcohol comprises directly compressible (DC) grade sugar alcohol.

In an embodiment of the invention, the directly compressible (DC) grade sugar alcohol comprises sugar alcohol selected from the group consisting of xylitol, maltitol, mannitol, erythritol, isomalt, sorbitol, lactitol, and any mixture thereof.

Sorbitol is in directly compressible (DC) grade from nature.

In an advantageous embodiment of the invention, the pouch composition comprises at least one further sugar alcohol.

In an embodiment of the invention, the pouch composition comprises the at least one further sugar alcohol in an amount of at least 1% by weight of the composition, such as at least 2% by weight of the composition, such as at least 5% by weight of the composition, such as at least 10% by weight of the composition, such as at least 15% by weight of the composition.

In an embodiment of the invention, the pouch composition comprises the at least one further sugar alcohol in an amount of 1 to 50% by weight of the composition, such as 2 to 70% by weight of the composition, such as 5 to 40% by weight of the composition, such as 10 to 50% by weight of the composition, such as 15 to 30% by weight of the composition.

In an advantageous embodiment of the invention, the at least one further sugar alcohol comprises directly compressible (DC) grade sugar alcohol.

In an embodiment of the invention, the at least one further sugar alcohol is directly compressible (DC) grade sugar alcohol.

In an embodiment of the invention, the at least one sugar alcohol comprises non-directly compressible (non-DC) grade sugar alcohol(s) and the at least one further sugar alcohol comprises directly compressible (DC) grade sugar alcohol.

In an embodiment of the invention, the non-directly compressible (non-DC) grade sugar alcohol is selected from the list consisting of xylitol, maltitol, mannitol, erythritol, isomalt, lactitol, and mixtures thereof.

Sugar alcohols may advantageously facilitate and induce salivation of the pouch composition, whereby release of nicotine is obtained, such as of nicotine from the pouch.

Sugar alcohols may advantageously be used to further increase the nicotine release from the pouch.

Also, sugar alcohols may advantageously be used for obtaining a desirable mouthfeel by increasing salivation and thereby counteract any local dehydration or oral dehydrating sensation experienced by the user of the pouch.

In an embodiment of the pouch composition comprises non-directly compressible (non-DC) grade sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the pouch composition comprises non-directly compressible (non-DC) grade sugar alcohol in an amount of 1 to 80% by weight of the composition, such as 10 to 70% by weight of the composition, such as 10 to 60% by weight of the composition, such as 15 to 60% by weight of the composition, such as 20 to 60% by weight of the composition, such as 20 to 50% by weight of the composition.

In an embodiment of the invention, the at least one sugar alcohol comprises sugar alcohol selected from the list consisting of maltitol, mannitol, erythritol, isomalt, sorbitol, lactitol, and any combination thereof.

In an embodiment of the invention the at least one sugar alcohol comprises sugar alcohol selected from the list consisting of maltitol, mannitol, isomalt, sorbitol, lactitol, and any combination thereof.

In an embodiment of the invention the at least one sugar alcohol comprises sugar alcohol selected from the list consisting of maltitol, mannitol, sorbitol, lactitol, and any combination thereof.

In an embodiment of the invention the pouch composition is free of DC-grade xylitol.

In an embodiment of the invention the pouch composition is free of xylitol.

In an embodiment of the invention the pouch composition is free of DC-grade sugar alcohol.

In an embodiment of the invention, the pouch composition comprises sugar alcohol in an amount of at least 14.0 % by weight of the pouch composition.

In an embodiment of the invention, the pouch composition comprises at least one non-wheat fiber.

In an embodiment of the invention, the pouch composition comprises at least one non-sodium carbonate pH regulating agent.

In an embodiment of the invention, the pouch composition comprises pH regulating agent in an amount of more than 5.5% by weight of the pouch composition.

In an embodiment of the invention, the pouch composition comprises sodium chloride.

In an embodiment of the pouch composition comprises at least two non-direct compressible (non-DC) grade sugar alcohols selected from the group consisting of xylitol, maltitol, mannitol, erythritol, isomalt, lactitol, and any combination thereof.

In an advantageous embodiment of the invention, the non-directly compressible (non-DC) grade sugar alcohol(s) is provided as particles, which have not been subject to granulation or agglomeration steps.

The terminology directly compressible is well-known within the art of tableting, i.e. in technical field of compression of particles in a gathered compressed tablet. Directly compressible is routinely referred to as DC by many manufactures of such particles.

In an embodiment of the invention the at least one non-directly compressible (non-DC) grade sugar alcohol are defined as non-DC grade with reference to the Compressibility Index according to European Pharmacopeia 6.0 and where particles consisting of the non-DC grade sugar alcohol are having a compressibility index which is greater than 21%.

In an embodiment of the invention the at least one non-directly compressible (non-DC) grade sugar alcohol are defined as non-DC with reference to the Compressibility Index according to European Pharmacopeia 6.0 and where particles consisting of the non-DC grade sugar alcohol are having a compressibility index which is greater than 21% and less than 37%.

In an embodiment of the invention the pouch composition comprises a further sugar alcohol, wherein said further sugar alcohol is directly compressible (DC).

In an embodiment of the invention said further sugar alcohol is defined as DC with reference to the Compressibility Index according to European Pharmacopeia 6.0 and where particles consisting of said further sugar alcohol are having a compressibility index which is less than 21%, such as less than 15%, such as less than 10%.

In an advantageous embodiment of the invention, the pouch composition comprises nicotine in an amount of at least 0.1% by weight, such as least 0.2% by weight of the pouch composition.

In an embodiment of the invention, the pouch composition comprises nicotine in an amount of 0.1 to 5.0% by weight of the pouch composition, such as 0.2 to 4.0% by weight of the pouch composition, such as 1.0 to 2.0% by weight of the pouch composition.

In an advantageous embodiment of the invention, the pouch composition comprises nicotine selected from the group consisting of a nicotine salt, nicotine free base, nicotine-ion exchange resin combination, a nicotine inclusion complex or nicotine in any non-covalent binding; nicotine bound to zeolites; nicotine bound to cellulose, such as microcrystalline cellulose, or starch microspheres, and mixtures thereof.

In an advantageous embodiment of the invention, the pouch composition comprises nicotine-ion exchange resin combination in an amount corresponding to 0.1 to 20% by weight of the pouch composition.

In an advantageous embodiment of the invention, the nicotine-ion exchange resin combination comprises nicotine complexed with ion exchange resin.

In an advantageous embodiment of the invention, the nicotine-ion exchange resin combination is nicotine complexed with ion exchange resin.

Thus, in the above embodiment the nicotine-ion exchange resin combination consists of nicotine complexed with ion exchange resin.

In an advantageous embodiment of the invention, the nicotine-ion exchange resin combination comprises free-base nicotine mixed with ion exchange resin.

One advantage of the above embodiment may be providing sustained release of nicotine. At the same time, the release rate of nicotine is not too slow to give the user the craving relief desired.

In an advantageous embodiment of the invention, the nicotine-ion exchange resin combination comprises nicotine in an amount of between 5 and 50% by weight.

In an embodiment of the invention the nicotine-ion exchange resin combination comprises nicotine complexed with ion exchange resin, wherein the nicotine constitutes an amount of between 5 and 50% by weight of nicotine-ion exchange resin combination.

In an embodiment of the invention the nicotine-ion exchange resin combination consists of nicotine complexed with ion exchange resin, wherein the nicotine constitutes an amount of between 10 and 50% by weight of nicotine-ion exchange resin combination, such as between 10 and 40% by weight of nicotine-ion exchange resin combination, such as. between 10 and 30% by weight of nicotine-ion exchange resin combination, such as between 10 and 25% by weight of nicotine-ion exchange resin combination.

In an embodiment of the invention the nicotine-ion exchange resin combination comprises free-base nicotine mixed with ion exchange resin, wherein the nicotine constitutes an amount of between 5 and 50% by weight of nicotine-ion exchange resin combination.

In an embodiment of the invention the nicotine-ion exchange resin combination comprises free-base nicotine mixed with ion exchange resin, wherein the nicotine constitutes an amount of between 5 and 50% by weight of nicotine-ion exchange resin combination, such as .between 10 and 50% by weight of nicotine-ion exchange resin combination, such as .between 20 and 50% by weight of nicotine-ion exchange resin combination, such as between 25 and 50% by weight of nicotine-ion exchange resin combination, such as between 25 and 45% by weight of nicotine-ion exchange resin combination.

In an embodiment of the invention the nicotine-ion exchange resin combination comprises free-base nicotine mixed with ion exchange resin, wherein the nicotine constitutes an amount of between 5 and 40% by weight of nicotine-ion exchange resin combination, such as between 10 and 40% by weight of nicotine-ion exchange resin combination, such as between 10 and 35% by weight of nicotine-ion exchange resin combination, such as between 10 and 25% by weight of nicotine-ion exchange resin combination, such as between 10 and 15% by weight of nicotine-ion exchange resin combination.

In an advantageous embodiment of the invention, the nicotine-ion exchange resin combination comprises nicotine in an amount of between 5 and 50% by weight and ion-exchange resin in an amount between 10 and 95% by weight.

In an embodiment of the invention the nicotine-ion exchange resin combination comprises nicotine in an amount of between 5 and 50% by weight and ion-exchange resin in an amount between 10 and 95% by weight.

In an embodiment of the invention the nicotine-ion exchange resin combination comprises nicotine in an amount of between 10 and 30% by weight and ion-exchange resin in an amount between 20 and 90% by weight.

In an embodiment of the invention the nicotine-ion exchange resin combination consist of nicotine in an amount of between 10 and 30% by weight and ion-exchange resin in an amount between 70 and 90% by weight.

In an embodiment of the invention the nicotine-ion exchange resin combination is substantially free of water.

In an embodiment of the invention the nicotine-ion exchange resin combination further comprises glycerol complexed with the ion-exchange resin.

Glycerol may be added with the purpose of reducing dustiness of the nicotine-ion exchange resin combination.

In an embodiment of the invention, the nicotine-ion exchange resin combination further comprises glycerol in an amount of 0.1 to 50% by weight, such as 5 to 40% by weight, such as 5 to 30% by weight.

In an embodiment of the invention the nicotine-ion exchange resin combination comprises nicotine in an amount of between 5 and 50% by weight and ion-exchange resin in an amount between 20 and 75% by weight.

In an embodiment of the invention the nicotine-ion exchange resin combination comprises water in an amount of no more than 75% by weight, such as no more than 50% by weight, such as no more than 40% by weight, such as no more than 30% by weight, such as no more than 20% by weight, such as no more than 10% by weight, such as no more than 5% by weight.

In an advantageous embodiment of the invention, the ion exchange resin comprises one or more resin(s) selected from the group consisting of:
(i) a methacrylic, weakly acidic type of resin containing carboxylic functional groups,
(ii) a copolymer of methacrylic acid and divinylbenzene, said copolymer containing carboxylic functional groups,
(iii) a polystyrene, strongly acidic type of resin containing sulphonic functional groups,
(iv) a polystyrene, intermediate acidic type of resin containing phosphonic functional groups, and
(v) a combination thereof.

In an advantageous embodiment of the invention, the ion exchange resin comprises polacrilex resin.

In an advantageous embodiment of the invention, the ion exchange resin is polacrilex resin.

In an embodiment of the invention, the polacrilex resin comprises or is Amberlite^{®}IRP64.

In an advantageous embodiment of the invention, the pouch composition comprises water in an amount of 15-65% by weight of the composition, such as 15-60% by weight of the composition, such as 15-50% by weight of the composition, such as 20-50% by weight of the composition, such as 20-40% by weight of the composition.

In an embodiment of the invention, the pouch composition comprises water in an amount of 15-65% by weight of the composition, such as 20-65% by weight of the composition, such as 25-65% by weight of the composition.

In an embodiment of the invention, the pouch composition comprises water in an amount of 15-65% by weight of the composition, such as 15-60% by weight of the composition, such as 15-50% by weight of the composition, such as 15-40% by weight of the composition.

In an embodiment of the invention, the pouch composition comprises water in an amount of 15-60% by weight of the composition, such as 15-50% by weight of the composition, such as 15-40% by weight of the composition, such as 15-30% by weight of the composition.

In an embodiment of the invention, the pouch composition comprises water in an amount of 15-40% by weight of the composition.

The water may be added as a separate component to be fully or partly mixed into other components, such as fibers. E.g. when adding a nicotine ion-exchange resin combination consisting of a mixture of free base nicotine with ion exchange resin and water, a significant amount of water of the final pouch composition may come from this mixture. For example, if the final amount pouch composition comprises 5% water from the nicotine-ion exchange resin combination, then up to one third of the water in the pouch composition derives from the nicotine-ion exchange resin combination.

In an advantageous embodiment of the invention, the pouch composition comprises at least one water-insoluble fiber.

In an advantageous embodiment of the invention, the pouch composition comprises at least one water-insoluble fiber in an amount between 5 and 50 % by weight of the pouch composition, such as 10-45% by weight of the pouch composition, such as 15-40% by weight of the pouch composition.

In an embodiment of the invention, the pouch composition comprises at least one water-insoluble fiber in an amount between 5 and 50 % by weight of the pouch composition, such as 5-45% by weight of the pouch composition, such as 5-40% by weight of the pouch composition.

In an embodiment of the invention, the pouch composition comprises at least one water-insoluble fiber in an amount between 5 and 50 % by weight of the pouch composition, such as 10-50% by weight of the pouch composition, such as 15-50% by weight of the pouch composition.

An advantage of the above embodiment may be that a residue is left even after use of a nicotine pouch comprising the pouch composition. This may lead to a pleasant perception for users of the nicotine pouch, e.g. due to similarity with tobacco containing products.

The water-insoluble fiber may advantageously provide a desirable mouthfeel throughout the use of the pouch.

A further advantage of the invention is that a very attractive soft, moist, and moldable texture and mouthfeel is obtained due to a combination of sugar alcohol, water-insoluble fiber and water. The desirable texture and mouthfeel may be obtained while still being able to store manufactured pouches together in abutment e.g. in cans etc. without sticking too much together to result in ruptures of the pouches when being removed.

In an advantageous embodiment of the invention, the water-insoluble fiber is a non-tobacco fiber.

In an advantageous embodiment of the invention, the water-insoluble fiber is a plant fiber.

In an advantageous embodiment of the invention, the water-insoluble fiber is selected from wheat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, cellulose fibers, bran fibers, bamboo fibers, powdered cellulose, and combinations thereof.

Powdered cellulose within the scope of the invention is understood to be cellulose prepared by processing alpha-cellulose obtained as a pulp from strains of fibrous plant materials, such as wood pulp.

In an embodiment of the invention, the water-insoluble fiber comprises or consists of cereal fibers.

In an embodiment of the invention, the water-insoluble fiber comprises or consists of fruit and/or vegetable fibers.

In an embodiment of the invention, the water-insoluble composition comprises or consists of water-insoluble fiber selected from wheat fibers, oat fibers, pea fibers, powdered cellulose, or combinations thereof.

In an embodiment of the invention, the water-insoluble composition comprises or consists of water-insoluble fiber selected from wheat fibers, oat fibers, pea fibers, or combinations thereof.

In an embodiment of the invention, the water-insoluble composition comprises or consists of water-insoluble fiber selected from wheat fibers, oat fibers, or combinations thereof.

Non-limiting examples of usable water-insoluble fibers include Vitacel WF 600, Vitacel HF 600, Vitacel P95, Vitacel WF 200, Vitacel L00, Vitacel Erbsenfaser EF 150, Vitacel bamboo fiberbaf 90, Vitacel HF 600, Vitacel Cellulose L700G, Vitacel PF200, Vitacel potatofiber KF200, Vitacel bamboo fiberhaf BAF40, Vitacel Haferfaser/oat fiber HF-401-30 US.

Non-limiting examples of usable powdered cellulose include Vitacel L 00, Vitacel Cellulose L700G, Vitacel LC1000, Vitacel L600-20, Vitacel L600 etc.

In an embodiment, the powdered cellulose is chemically unmodified. Thus, powdered cellulose may be chemically unmodified cellulose fibers, which do not include e.g. microcrystalline cellulose (MCC).

In an advantageous embodiment of the invention, the water-insoluble fiber has a water binding capacity of at least 200%, such as at least 300%, such as at least 400%.

An advantage of the above embodiment may be that the high water-binding capacity enables pouch compositions having a high water-content.

Furthermore, the pouches having a high water-content where found to have a desirable texture and mouthfeel while it is still possible to store the manufactured pouches together in abutment e.g. in cans etc. without sticking too much together to result in ruptures of the pouches when being removed.

Also, water-insoluble fibers having a high water-binding capacity may reduce any nicotine exchange induced by the divalent cations happening prior to the pouch being used.

Hence, pouches comprising water-insoluble fibers having a high water-binding capacity could advantageously decrease the relative standard deviation (RSD) on the nicotine content.

In an advantageous embodiment of the invention, the content of nicotine between a series of at least 10 oral pouches comprising said pouch composition holds a relative standard deviation (RSD) below 10%, preferably below 8%, more preferably at most 6%, even more preferably at most 4%, most preferably at most 2%.

In an embodiment of the invention, the content of the nicotine between a series of at least 10 oral pouches comprising said pouch composition holds a relative standard deviation (RSD) of 0.1 - 10%, preferably 0.1 - 8%, more preferably 0.1 - 6%, even more preferably 0.1 - 4%, and most preferably 0.1 - 2%.

In an embodiment of the invention, the water-insoluble fiber has a water binding capacity of 300 to 1500%, such as 400 to 1300%.

In an embodiment of the invention, the water-insoluble fiber has a water binding capacity of 200% to 1500%, such as 300 to 1300%, such as 200 to 800%, such as 300 to 800%, such as 400 to 600%.

In an embodiment of the invention, the water-insoluble fiber has a water binding capacity of 200 to 1500%, such as 300 to 1300%, such as 300 to 900%, such as 300 to 700%, such as 400 to 700%.

In an embodiment of the invention, the water-insoluble fiber has a water binding capacity of 200 to 1500%, such as 400 to 1500%, such as 500 to 1500%, such as 500 to 1200%, such as 500 to 1000%.

In an embodiment of the invention, the water-insoluble fiber has a swelling capacity of at least 5.0 mL/g, such as 5.0 - 20 mL/g.

An advantage of the above embodiment is that the amount of water-insoluble fiber can be reduced without compromising the mouthfeel during use. If an amount of water-insoluble fiber is substituted for a water-soluble component, the swelling of the water-insoluble fiber will during use counteract the dissolution of the water-soluble component, thereby the user will not experience any decrease in pouch content during use.

In an embodiment of the invention, the water-insoluble fibers are selected from pea fibers, powdered cellulose, and combinations thereof, and wherein the pouch composition comprises flavor in an amount of no more than 10% by weight of the pouch composition.

In an embodiment of the invention, the pouch composition comprises water-insoluble fibers selected from pea fibers and powdered cellulose, or a combination thereof, and flavor in an amount of 0.01 - 10% by weight of the pouch composition.

In an advantageous embodiment of the invention, the water-insoluble fiber has a density of 50 to 500 gram per Liter, such as 100 to 400 gram per Liter, such as 200 to 300 gram per Liter.

The use of water-insoluble fiber having a relatively low bulk density, will provide not only a good mouthfeel, but also an effective release from the pouch, due to the fact that a relatively low bulk density promotes effective salivation, thereby dissolution and release of water-soluble ingredients of the composition.

In an advantageous embodiment of the invention, the pouch composition comprises a pH regulating agent.

In an advantageous embodiment of the invention, the pouch composition comprises pH regulating agent in an amount between 0.01 and 15% by weight of the pouch composition, such as between 0.5 and 10% by weight of the pouch composition, such as between 1 and 10% by weight of the pouch composition, such as between 5 and 10% by weight of the pouch composition.

Obtaining a relatively fast release rate of nicotine and an effective uptake/absorption may be desirable as this ensures a fast effect for the user, i.e. craving relief.

Furthermore, the combination of having an effective release and an effective absorption advantageously enables a relative high exploitation of the nicotine dose within the pouch. Having a relative high exploitation of the nicotine dose within the pouch may further provide a reduction of necessary nicotine dose of the pouch, without compromising the resulting effect. A lower nicotine dose may in tern result in a reduction in production cost, as nicotine may be relatively expensive, but may also assist users who want to lower their intake of nicotine.

In an advantageous embodiment of the invention, the pH regulating agent is a basic pH regulating agent, such as a basic buffering agent.

In an advantageous embodiment of the invention, the pH regulating agent is a buffering agent, such as a basic buffering agent.

In an embodiment of the invention, the pouch composition is adapted to give a pH of at least 8.0, such as a pH of at least 9.0, when 2.0 gram of pouch composition is added to 20 mL of 0.02 M potassium dihydrogen phosphate-buffer (pH adjusted to 7.4).

An advantage of the above embodiment may be that a relatively effective uptake of nicotine is facilitated due to the high pH value obtained.

A further advantage of the above embodiment may be that the need for preservative may be decreased or even eliminated and that low amounts of such preservatives may be used if not absent.

Also, the high pH value obtained may advantageously provide for a tingling sensation in the mouth which may be perceived as a desirable mouthfeel, e.g. due to resemblance with tobacco-based pouch products.

In an embodiment of the invention, the pH regulating agent is selected from the group consisting of Acetic acid, Adipic acid, Citric acid, Fumaric acid, Glucono-δ-lactone, Gluconic acid, Lactic acid, Malic acid, Maleic acid, Tartaric acid, Succinic acid, Propionic acid, Ascorbic acid, Phosphoric acid, Sodium orthophosphate, Potassium orthophosphate, Calcium orthophosphate, Sodium diphosphate, Potassium diphosphate, Calcium diphosphate, Pentasodium triphosphate, Pentapotassium triphosphate, Sodium polyphosphate, Potassium polyphosphate, Carbonic acid, Sodium carbonate, Sodium bicarbonate, Potasium carbonate, Calcium carbonate, Magnesium carbonate, Magnesium oxide, or any combination thereof.

In an advantageous embodiment of the invention, the pH regulating agent is selected from the group consisting Sodium carbonate, Sodium bicarbonate, Potassium carbonate, and Magnesium carbonate; Potassium bicarbonate; trometamol; phosphate buffer, or any combination thereof.

In the present context the term trometamol refer to (tris(hydroxymethyl)aminomethane), also sometimes referred to as tris buffer.

In an embodiment of the invention, the pouch composition further comprises inorganic multivalent cations, such as inorganic divalent cations.

In an embodiment of the invention, the pouch composition further comprises inorganic multivalent cations selected from the group consisting of multivalent ions of calcium, magnesium, zinc, aluminum, barium, iron, manganese, copper, lead, cobalt, nickel, such as Ca2+, Mg2+, Zn2+, Al3+, Ba2+, Fe2+, Fe3+, Fe4+, Mn2+, Mn4+, Cu4+, or any combinations thereof, or from the group consisting of Ca2+, Mg2+, and any combination thereof.

In an advantageous embodiment of the invention, the pouch composition is adapted to release at least 30% nicotine within 10 minutes when exposed to *in vitro* conditions described in example 6A.

In an advantageous embodiment of the invention, the pouch composition comprises sodium chloride in an amount of 0.0-3.0% by weight of the pouch compositions, such as 0.05 - 1.0% by weight of the pouch composition, such as 0.1 - 1.0% by weight of the pouch composition.

NaCl may advantageously be added in small amounts, i.e. 0.0-3.0% by weight as a flavor enhancer. Adding higher amounts of NaCl could induce an undesirable taste or mouthfeel.

In an advantageous embodiment of the invention, the pouch composition further comprises a preservative.

The preservative may help to preserve the pouch composition against undesirable microbiological growths.

In an advantageous embodiment of the invention, the pouch composition further comprises a preservative in an amount of 0.05 to 0.5% by weight of the pouch composition, such as 0.1 to 0.2% by weight of the pouch composition.

Non-limiting examples of usable preservatives within the scope of the invention includes sorbic acid (E200) and salts thereof (e.g. sodium sorbate (E201), potassium sorbate (E202), calcium sorbate (E203)), benzoic acid (E210) and salts thereof (e.g. sodium benzoate (E211), potassium benzoate (E212), calcium benzoate (E213)).

In an advantageous embodiment of the invention, the pouch composition comprises less than 0.1% by weight of preservatives, such as less than 0.05% by weight of preservatives.

Thus, the pouch composition may comprise preservatives in an amount of 0 to 0.1 % by weight of preservatives, such as in an amount of 0 to 0.05% by weight of preservatives. This includes zero content of preservatives, i.e. that the pouch composition is free of preservatives. The low amount or even absence of preservative may be realized by obtaining a relatively alkaline environment, such as by the use of free-base nicotine or by the use of alkaline buffering agents.

In an advantageous embodiment of the invention, the pouch composition is free of preservatives.

In an advantageous embodiment of the invention, the pouch composition is a non-tobacco pouch composition.

In an advantageous embodiment of the invention, the pouch composition comprises less than 2.0% by weight of tobacco, such as less than 1.0% by weight of tobacco, such as less than 0.5% by weight of tobacco, such as 0.0% by weight of tobacco.

In an embodiment of the invention, the pouch composition is free of humectants consisting of alginate, propylene glycol, hydroxypropyl cellulose, glycerol, modified starch, triacetin, polyethylene glycol (PEG), pectin, and xanthan gum and the pouch composition comprises water in an amount of 15-65% by weight of the composition, such as 15-60% by weight of the composition, such as 15-50% by weight of the composition, such as 20-50% by weight of the composition, such as 20-40% by weight of the composition.

In an embodiment of the invention, the pouch composition is free of humectants and the pouch composition comprises water in an amount of 15-65% by weight of the composition, such as 15-60% by weight of the composition, such as 15-50% by weight of the composition, such as 20-50% by weight of the composition, such as 20-40% by weight of the composition.

In an embodiment of the invention, the pouch composition comprises the at least one sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the invention, the pouch composition comprises the at least one sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition, and the at least one sugar alcohol comprises non-directly compressible (non-DC) grade sugar alcohol selected from the list consisting of xylitol, maltitol, mannitol, erythritol, isomalt, lactitol, and any combination thereof.

In an embodiment of the invention, the pouch composition comprises the at least one sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition and the at least one sugar alcohol comprises non-directly compressible (non-DC) grade sugar alcohol in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the invention, the at least one sugar alcohol comprises non-directly compressible (non-DC) grade sugar alcohol selected from the list consisting of xylitol, maltitol, mannitol, erythritol, isomalt, lactitol, and any combination thereof and in an amount of 1 to 80% by weight of the composition, such as 2 to 70% by weight of the composition, such as 5 to 60% by weight of the composition, such as 10 to 50% by weight of the composition, such as 15 to 50% by weight of the composition.

In an embodiment of the invention, the pouch composition comprises nicotine-ion exchange resin combination in an amount corresponding to 0.1 to 20% by weight of the pouch composition and the nicotine-ion exchange resin combination comprises nicotine complexed with ion exchange resin.

In an embodiment of the invention, the pouch composition comprises nicotine-ion exchange resin combination in an amount corresponding to 0.1 to 20% by weight of the pouch composition and the nicotine-ion exchange resin combination is nicotine complexed with ion exchange resin.

In an embodiment of the invention, the pouch composition comprises nicotine-ion exchange resin combination in an amount corresponding to 0.1 to 20% by weight of the pouch composition and the nicotine-ion exchange resin combination comprises free-base nicotine mixed with ion exchange resin.

In an embodiment of the invention, the pouch composition comprises nicotine-ion exchange resin combination in an amount corresponding to 0.1 to 20% by weight of the pouch composition and the nicotine-ion exchange resin combination is free-base nicotine mixed with ion exchange resin.

In an embodiment of the invention, the pouch composition comprises nicotine-ion exchange resin combination in an amount corresponding to 0.1 to 20% by weight of the pouch composition and wherein the nicotine-ion exchange resin combination comprises nicotine in an amount of between 5 and 50% by weight.

In an embodiment of the invention, the pouch composition comprises nicotine-ion exchange resin combination in an amount corresponding to 0.1 to 20% by weight of the pouch composition and wherein the nicotine-ion exchange resin combination comprises nicotine in an amount of between 5 and 50% by weight and ion-exchange resin in an amount between 10 and 95% by weight.

In an embodiment of the invention, the pouch composition comprises nicotine-ion exchange resin combination in an amount corresponding to 0.1 to 20% by weight of the pouch composition, wherein the nicotine-ion exchange resin combination comprises nicotine in an amount of between 5 and 50% by weight and wherein the ion-exchange resin comprises one or more resin(s) selected from the group consisting of:
(i) a methacrylic, weakly acidic type of resin containing carboxylic functional groups,
(ii) a copolymer of methacrylic acid and divinylbenzene, said copolymer containing carboxylic functional groups,
(iii) a polystyrene, strongly acidic type of resin containing sulphonic functional groups,
(iv) a polystyrene, intermediate acidic type of resin containing phosphonic functional groups, and
(v) a combination thereof.

In an embodiment of the invention, the pouch composition comprises nicotine-ion exchange resin combination in an amount corresponding to 0.1 to 20% by weight of the pouch composition, wherein the nicotine-ion exchange resin combination comprises nicotine in an amount of between 5 and 50% by weight and ion-exchange resin in an amount between 10 and 95% by weight and wherein the ion-exchange resin comprises one or more resin(s) selected from the group consisting of:
(i) a methacrylic, weakly acidic type of resin containing carboxylic functional groups,
(ii) a copolymer of methacrylic acid and divinylbenzene, said copolymer containing carboxylic functional groups,
(iii) a polystyrene, strongly acidic type of resin containing sulphonic functional groups,
(iv) a polystyrene, intermediate acidic type of resin containing phosphonic functional groups, and
(v) a combination thereof.

In an embodiment of the invention, the pouch composition comprises nicotine-ion exchange resin combination in an amount corresponding to 0.1 to 20% by weight of the pouch composition, wherein the nicotine-ion exchange resin combination comprises nicotine in an amount of between 5 and 50% by weight and wherein the ion exchange resin is polacrilex resin.

In an embodiment of the invention, the pouch composition comprises nicotine-ion exchange resin combination in an amount corresponding to 0.1 to 20% by weight of the pouch composition, wherein the nicotine-ion exchange resin combination comprises nicotine in an amount of between 5 and 50% by weight and ion-exchange resin in an amount between 10 and 95% by weight and wherein the ion exchange resin is polacrilex resin.

In an embodiment of the invention, the pouch composition comprises water in an amount of 15-65% by weight of the composition, such as 15-60% by weight of the composition, such as 15-50% by weight of the composition, such as 20-50% by weight of the composition, such as 20-40% by weight of the composition and nicotine in an amount of 0.1 to 5.0% by weight of the pouch composition, such as 0.2 to 4.0% by weight of the pouch composition, such as 1.0 to 2.0% by weight of the pouch composition.

In an embodiment of the invention, the pouch composition comprises water in an amount of 15-65% by weight of the composition, such as 15-60% by weight of the composition, such as 15-50% by weight of the composition, such as 20-50% by weight of the composition, such as 20-40% by weight of the composition, nicotine in an amount of 0.1 to 5.0% by weight of the pouch composition, such as 0.2 to 4.0% by weight of the pouch composition, such as 1.0 to 2.0% by weight of the pouch composition and at least one water-insoluble fiber in an amount between 5 and 50 % by weight of the pouch composition, such as 10-45% by weight of the pouch composition, such as 15-40% by weight of the pouch composition.

In an embodiment of the invention, the pouch composition comprises at least one water-insoluble fiber in an amount between 5 and 50 % by weight of the pouch composition, such as 10-45% by weight of the pouch composition, such as 15-40% by weight of the pouch composition, wherein the water-insoluble fiber is selected from wheat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, cellulose fibers, bran fibers, bamboo fibers, powdered cellulose, and combinations thereof.

In an embodiment of the invention, the pouch composition comprises pH regulating agent in an amount between 0.01 and 15% by weight of the pouch composition, such as between 0.5 and 10% by weight of the pouch composition, such as between 1 and 10% by weight of the pouch composition, such as between 5 and 10% by weight of the pouch composition, and the pH regulating agent is selected from the group consisting Sodium carbonate, Sodium bicarbonate, Potassium carbonate, and Magnesium carbonate; Potassium bicarbonate; trometamol; phosphate buffer, or any combination thereof.

In an embodiment of the invention, the at least one sugar alcohol comprises non-directly compressible (non-DC) grade sugar alcohol selected from the list consisting of xylitol, maltitol, mannitol, erythritol, isomalt, lactitol, and any combination thereof. In an embodiment of the invention, the at least one sugar alcohol comprises non-directly compressible (non-DC) grade sugar alcohol selected from the list consisting of xylitol, maltitol, mannitol, erythritol, isomalt, lactitol, and any combination thereof, the non-directly compressible (non-DC) grade sugar alcohol(s) being provided as particles, which have not been subject to granulation or agglomeration steps.

The invention further relates to an oral pouched nicotine product comprising a saliva-permeable pouch and the pouch composition of the invention or any of its embodiments enclosed in said pouch.

In an advantageous embodiment of the invention, the pouched nicotine product comprises nicotine in an amount of 0.5 to 20 mg, such as 1.0 to 20 mg, such as 5.0 to 15 mg.

In an advantageous embodiment of the invention, the pouched nicotine product comprises nicotine-ion exchange resin combination in an amount of 1 to 100 mg.

In an embodiment of the invention the pouched nicotine product comprises nicotine-ion exchange resin combination in an of 1 to 100 mg, such as 20 to 100 mg, such as, 40 to 100 mg, such as 60 to 100 mg, such as 60 to 85 mg.

In an embodiment of the invention the pouched nicotine product comprises nicotine-ion exchange resin combination in an of 1 to 100 mg, such as 5 to 60 mg, such as, 10 to 50 mg, such as 10 to 40 mg, such as 25 to 40 mg.

In an embodiment of the invention, the pouched nicotine product is free of tobacco.

### DETAILED DESCRIPTION

As used herein the term "pouch composition" refers to the composition for use in an oral pouch, i.e. in pouches for oral use. Also, the terms "pouch composition" and "nicotine pouch composition" is used interchangeably.

As used herein the term "pouch" is intended to mean a container typically formed by a web of a fibrous material enclosing a cavity. The pouch is pouch designed for administration of an active ingredient in the oral cavity, and thus it is adapted for oral use, it is non-toxic and not water-soluble. The fibrous material may e.g. form a woven or non-woven web or fabric. The pouch may for example be sealed by bonding two corresponding pieces of web or fabric to each other along their edges to form a cavity for the nicotine and the non-water-soluble composition. In order to release the nicotine, the pouch is made water-permeable so as to allow saliva from the oral cavity to penetrate the pouch and enter the cavity, where the saliva can come into contact with the nicotine, whereby the nicotine are released from the oral pouch.

As used herein the term "humectant" is understood as a moistening agent used to keep pouches moist, i.e. a humectant is added to the pouch composition with the purpose of keeping the pouch moist. Hence, the term humectant does not refer to substances added for other purposes, hereunder also hygroscopic substances added for other purposes, such as sugar alcohols, water-insoluble fibers and glycerol associated with ion-exchange resin in nicotine-ion exchange resin combinations, such as nicotine polacrilex. Examples of humectants include alginate, propylene glycol, hydroxypropyl cellulose, and glycerol. It is noted that when glycerol is included as a humectant, the glycerol is added as free glycerol and therefore liquid at room temperature. Further examples of humectants include triacetin, modified starch, pectin, xanthan gum, etc. The term humectant does not refer to sugar alcohols comprising 4 or more carbons. Also, the term humectant does not refer to fibers, such as water-insoluble fiber, such as wheat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, cellulose fibers, bran fibers, bamboo fibers, powdered cellulose, and combinations thereof. Also, the term humectant does not include e.g. NaCl.

As used here, a non-tobacco pouch composition refers to a non-tobacco based composition. In an embodiment of the invention, the non-tobacco pouch composition comprises at most 2% tobacco fibers, or is free of tobacco fibers.

As used herein the term "nicotine" refers to nicotine used as a refined/isolated substance. Nicotine may be isolated from tobacco and added to pouch compositions. Particularly, nicotine does not refer to tobacco materials having a content of nicotine. Thus, when referring to nicotine amounts also to be understood as the nicotine dose, the amounts refers to the amount of pure nicotine. Nicotine also covers nicotine not obtained from tobacco, often referred to as synthetic nicotine.

As used herein, the term "nicotine-ion exchange resin combination" refer to a combination comprising nicotine complexed with ion exchange resin and/or nicotine mixed with ion exchange resin.

As used herein, the term "nicotine complexed with ion-exchange resin" refers to nicotine bound to an ion exchange resin.

In the present context the term "free-base nicotine mixed with ion exchange resin" refers to a mixture comprising free-base nicotine and ion exchange resin. It is noted that even if some embodiments comprise a combination of nicotine complexed with ion exchange resin and nicotine in its free-base form mixed with ion exchange resin, the term "free-base nicotine mixed with ion exchange resin" requires the presence of nicotine in its free-base form. In some embodiments, the mixture is an aqueous mixture. Free-base nicotine and water is mixed with ion-exchange resin, whereby a mixture comprising both free-base nicotine and ion exchange resin is obtained. Free-base nicotine mixed with ion exchange resin is referred to as "premix" in the examples. As used herein the term "powder composition" refers to composition in the form of powder, i.e. as a particulate material having a relatively small particle size, for example between 1 and 1200 micrometer. Particularly, by powder composition is not meant a powdered tobacco.

As used herein the term "free-base nicotine" refers to non-protonated form of nicotine, and therefore does not include nicotine salts or nicotine provided as a complex between nicotine and an ion exchange resin. Nevertheless, the free-base nicotine may be mixed with an amount of ion exchange resin or water-soluble compositions such as sugar alcohols or water-soluble fibers. While free-base nicotine includes both free-base nicotine extracted from tobacco as well as synthetically manufactured free-base nicotine, the free-base nicotine is not provided in the form of tobacco or powdered tobacco. Typically, free-base nicotine is provided as a liquid.

As used herein the term "water-insoluble" refers to relatively low water-solubility, for example a water-solubility of less than 0.1 gram of water-soluble composition or substance per 100 mL of water measured at 25 degrees Celsius, atmospheric pressure and pH of 7.0. When referring to "insoluble", water-insoluble is meant unless otherwise stated.

As used herein the term "effective release" refers to the total release of nicotine over the release period of the experiment or the use period.

As used herein, the term "dissolve" is the process where a solid substance enters a solvent (such as oral saliva or water within the pouch) to yield a solution.

The pouches of the invention provide a nicotine release into the oral cavity. A release profile of nicotine may be obtained which both comprises a fast release period and a sustained release period.

As used herein the term "fast release" or "fast release period" may refer to the initial 2 minutes of the nicotine release profile, whereas the term "sustained release period refers" to the subsequent period of the release profile until end of experiment or end of use.

As used herein the term "fast release rate" refers to the released nicotine per minute within the initial 2 minutes.

In an embodiment of the invention the pouch composition comprises high intensity sweetener.

Preferred high intensity sweeteners include, but are not limited to sucralose, aspartame, salts of acesulfame, such as acesulfame potassium, alitame, saccharin and its salts, cyclamic acid and its salts, glycyrrhizin, dihydrochalcones, thaumatin, monellin, stevioside and the like, alone or in combination.

In an embodiment of the invention, the pouch composition comprises bulk sweeteners including sugar and/or sugarless components.

In an embodiment of the invention, the pouch composition comprises bulk sweetener in the amount of 1.0 to about 80% by weight of the pouch composition, more typically constitute 5 to about 70% by weight of the pouch composition, and more commonly 10 to 60% by weight of the pouch composition or 10-50% by weight of the pouch composition. In some embodiments, inclusion of certain ingredients may limit the about amounts of bulk sweetener further.

The sweeteners may often support the flavor profile of the pouch composition.

Sugar sweeteners generally include, but are not limited to saccharide-containing components commonly known in the art of pouches, such as sucrose, dextrose, maltose, saccharose, lactose, sorbose, dextrin, trehalose, D-tagatose, dried invert sugar, fructose, levulose, galactose, corn syrup solids, glucose syrup, hydrogenated glucose syrup, and the like, alone or in combination.

The sugar sweetener can be used in combination with sugarless sweeteners. Generally, sugarless sweeteners include components with sweetening characteristics but which are devoid of the commonly known sugars and comprise, but are not limited to, sugar alcohols comprising 4 or more carbons , such as sorbitol, mannitol, xylitol, hydrogenated starch hydrolyzates, maltitol, isomalt, erythritol, lactitol and the like, alone or in combination.

As used herein the term "flavor" is understood as having its ordinary meaning within the art. Flavor includes liquid and powdered flavors. Thus, flavors do of course not include sweeteners (such as sugar, sugar alcohols and high intensity sweeteners), or acids providing pure acidity/sourness, nor compounds providing pure saltiness (e.g. NaCl) or pure bitterness. Flavor enhancers include substances that only provide saltiness, bitterness or sourness. Flavor enhancers thus include e.g. sodium chloride, Citric acid, ammonium chloride etc.

The flavors can be natural or synthetic flavors.

In an embodiment of the invention the pouch composition comprises flavor. Flavor may typically be present in amounts between 0.01 and 15% by weight of the total composition of the pouch, such as between 0.01 and 5% by weight of the total composition.

Non-exhaustive examples of flavors suitable in embodiments of the present invention are coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

As used herein, the term "pH regulating agent" refers to agents, which active adjust and regulates the pH value of the solution to which they have been added or are to be added. Thus, pH regulating agents may be acids and bases, including acidic buffering agents and alkaline buffering agents. On the other hand, pH regulating agents does not including substances and compositions that can only affect the pH by dilution. Furthermore, pH regulating agents does not include e.g. flavoring, fillers, etc.

According to an embodiment of the invention, the enhancer comprises one or more pH-regulating agent, such as a buffering agent.

In an embodiment of the invention, said pH-regulating agents are selected from the group consisting of Acetic acid, Adipic acid, Citric acid, Fumaric acid, Glucono-δ-lactone, Gluconic acid, Lactic acid, Malic acid, Maleic acid, Tartaric acid, Succinic acid, Propionic acid, Ascorbic acid, Phosphoric acid, Sodium orthophosphate, Potassium orthophosphate, Calcium orthophosphate, Sodium diphosphate, Potassium diphosphate, Calcium diphosphate, Pentasodium triphosphate, Pentapotassium triphosphate, Sodium polyphosphate, Potassium polyphosphate, Carbonic acid, Sodium carbonate, Sodium bicarbonate, Potasium carbonate, Calcium carbonate, Magnesium carbonate, Magnesium oxide, or any combination thereof.

Typically, the pouches comprise openings, where the characteristic opening dimension is adapted to a characteristic dimension of the matrix composition so as to retain the matrix composition inside the pouch before use and/or to retain a part of the matrix composition, such as an water-insoluble composition, inside the pouch during use.

In order to obtain a pouch having suitable opening dimensions in view of the matrix composition to be used, the material for the pouch may be selected accordingly, e.g. comprising e.g. woven and/or non-woven fabric.

In other words, according to the various embodiments, the pouch forms a membrane allowing passage of saliva and prevents or inhibits passage of said matrix composition. The membrane of the pouch may be of any suitable material e.g. woven or non-woven fabric (e.g. cotton, fleece etc.), heat sealable non-woven cellulose or other polymeric materials such as a synthetic, semi-synthetic or natural polymeric material. An example of suitable pouch material is paper made of pulp and a small amount of wet strength agent. A material suitable for use must provide a semi-permeable membrane layer to prevent the powder or composition from leaving the bag or pouch during use. Suitable materials are also those that do not have a significant impact on the release of nicotine from the pouch.

The pouch composition is filled into pouches and is maintained in the pouch by a sealing. An ideal pouch is chemically and physically stable, it is pharmaceutically acceptable, it is insoluble in water, it is easy to fill with powder and seal, and it provides a semi-permeable membrane layer which prevent the powder from leaving the bag, but permit saliva and therein dissolved or sufficiently small suspended components from the pouch composition in the pouch, such as nicotine, to pass through said pouch.

The pouch may be placed in the oral cavity by the user. Saliva then enters into the pouch, and the nicotine and other components, which are soluble in saliva, start to dissolve and are transported with the saliva out of the pouch into the oral cavity, where the nicotine may be absorbed.

### EXAMPLES

### Example 1A - Preparation of pouches designed for administration of nicotine

The material of the pouches is heat sealable non-woven cellulose, such as long fiber paper. Pouches that are not in form of non-woven cellulose fabric may also be used according to the invention.

The powder is filled into pouches and is maintained in the pouch by a sealing.

### Example 1B - Preparation of pouches designed for administration of nicotine

The material of the pouches is manufactured using rayon fibers, such as viscose rayon staple fibers. The pouch membrane is heat sealed along its edges except for an opening in one end into an inner cavity formed by the pouch membrane.

The powder is filled into pouches and is maintained in the pouch by a sealing.

### Example 2: Preparation of nicotine premixes

A 60 liter planetary Bear Varimixer mixer was charged with water, and nicotine was weighed and added. The mixer was stirred at low speed for 1 minute at ambient temperature. Then ion exchange resin Amberlite ^{®} IRP64 was weighed and added to the mixer. The mixer was closed, stirred at high speed for 5 minutes, opened and scraped down, if necessary. Finally the mixer was stirred for further 5 minutes at high speed. The total process time was 20 minutes.

Thereby, mixtures of nicotine and cation exchange resin were produced from the constituents stated in the below tables.

### Premix I:

**Table 1. Ingredients used to manufacture nicotine premix I (5.7% nicotine). % water in obtained nicotine-resin composition: 71.4**

| Constituent | Amount (kg) | Amount (%) |
|---|---|---|
| Nicotine | 1.0 | 5.7 |
| Water | 12.5 | 71.4 |
| Resin | 4.0 | 22.9 |
| Total | 17.5 | 100.0 |

### Premix II:

**Table 2. Ingredients used to manufacture nicotine premix II (13.2% nicotine). % water in obtained nicotine-resin composition: 34.1.**

| Constituent | Amount (kg) | Amount (%) |
|---|---|---|
| Nicotine | 1.08 | 13.2 |
| Water | 2.80 | 34.1 |
| Resin | 4.32 | 52.7 |
| Total | 8.20 | 100.0 |

### Premix III:

**Table 3. Ingredients used to manufacture nicotine premix III (18.5% nicotine). % water in obtained nicotine-resin composition:7.5.**

| Constituent | Amount (kg) | Amount (%) |
|---|---|---|
| Nicotine | 1.08 | 18.5 |
| Water | 0.44 | 7.5 |
| Resin | 4.32 | 74.0 |
| Total | 5.84 | 100.0 |

### Premix IV:

**Table 4. Ingredients used to manufacture nicotine premix IV (10% nicotine). % water in obtained nicotine-resin composition: 50.0.**

| Constituent | Amount (kg) | Amount (%) |
|---|---|---|
| Nicotine | 1.08 | 10.0 |
| Water | 5.40 | 50.0 |
| Resin | 4.32 | 40.0 |
| Total | 10.8 | 100.0 |

### Premix V:

**Table 5. Ingredients used to manufacture nicotine premix V (20% nicotine). % water in obtained nicotine-resin composition: 31.5.**

| Constituent | Amount (kg) | Amount (%) |
|---|---|---|
| Nicotine | 1.78 | 20.0 |
| Water | 2.80 | 31.5 |
| Resin | 4.32 | 48.5 |
| Total | 8.90 | 100.0 |

### Premix VI:

**Table 6. Ingredients used to manufacture nicotine premix VI (30% nicotine). % water in obtained nicotine-resin composition: 27.5.**

| Constituent | Amount (kg) | Amount (%) |
|---|---|---|
| Nicotine | 3.05 | 30.0 |
| Water | 2.80 | 27.5 |
| Resin | 4.32 | 42.5 |
| Total | 10.17 | 100.0 |

### Premix VII

**Table 7. Ingredients used to manufacture nicotine premix VII (35% nicotine). % water in obtained nicotine-resin composition: 25.6.**

| Constituent | Amount (kg) | Amount (%) |
|---|---|---|
| Nicotine | 3.83 | 35.0 |
| Water | 2.80 | 25.6 |
| Resin | 4.32 | 39.4 |
| Total | 10.95 | 100.0 |

### Premix VIII:

**Table 8. Ingredients used to manufacture nicotine premix VIII (42% nicotine).. % water in obtained nicotine-resin composition: 22.8.**

| Constituent | Amount (kg) | Amount (%) |
|---|---|---|
| Nicotine | 5.15 | 42.0 |
| Water | 2.80 | 22.8 |
| Resin | 4.32 | 35.2 |
| Total | 12.27 | 100.0 |

### Example 3: Preparation of pouch compositions

Pouches are prepared comprising powdered compositions as outlined in table 9 - 21. The pouches are made as follows.

Fibers and water are mixed using a planetary Bear Varimixer mixer for 5 minutes. Then, the following ingredients were added subsequently under continuous mixing: first the nicotine (mixed for 2 minutes), then the remaining ingredients except liquid flavor and glidant if any (mixed for 2 minutes), then liquid flavor if any (mixed for 1 minute), then glidant if any (mixed for 1 minute). The total mixing time is 9-11 minutes.

### Example 4: Preparation of filled pouches

The final pouch composition is filled into pouches (target fill weight 500 mg powder per pouch). The pouch material of example 1A or 1B may be used. The powder is filled into pouches and is maintained in the pouch by a sealing.

### Example 5A: Pouches

The pouch compositions are prepared from the ingredients in table 9 using preparation method described in example 3.

The pouch compositions are filled into pouches as described in example 4 (pouch material of examples 1A was used, but 1B could also have been applied).

**Table 9: Pouch compositions.**

| **Pouches** | **P01** | **P02** | **P03** | **P04** | **C1** | **C2** | **C3** | **C4** |
|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg |
| Water content [wt%] | 27 | 25 | 20 | 15 | 10 | 27 | 27 | 27 |

| Raw material | Content in weight percent | | | | | | | |
|---|---|---|---|---|---|---|---|---|
| NPR (16%) | 12.1 | 12.1 | 12.1 | 12.1 | 12.1 | 12.1 | 12.1 | 12.1 |
| Non-DC xylitol | 18.9 | 20.9 | 30.9 | 40.9 | 45.9 | 16.9 | 16.9 | 16.9 |
| Purified water | 27 | 25 | 20 | 15 | 10 | 27 | 27 | 27 |
| Wheat fiber | 25 | 25 | 20 | 15 | 15 | 25 | 25 | 25 |
| Sodium alginate | - | - | - | - | - | 2.0 | - | - |
| Glycerol | - | - | - | - | - | - | 2.0 | - |
| Hydroxypropyl cellulose | - | - | - | - | - | - | - | 2.0 |
| Sodium carbonate | 5.0 | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 | 8.9 |
| High intensity sweetener | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 |
| Total | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5B: Pouches

The pouch compositions are prepared from the ingredients in table 10 using preparation method described in example 3.

The pouch compositions are filled into pouches as described in example 4 (pouch material of examples 1A was used, but 1B could also have been applied).

**Table 10:**

| **Pouches** | **P10** | **P11** | **P12** | **P13** | **C5** |
|---|---|---|---|---|---|
| Amount of nicotine | 9.6 mg | 9.6 mg | 9.6 mg | 9.6 mg | 9.6 mg |
| Water content [wt%] | 27 | 25 | 20 | 15 | 10 |

| **Raw material** | **Content in weight percent** | | | | |
|---|---|---|---|---|---|
| Premix VI | 6.4 | 6.4 | 6.4 | 6.4 | 6.4 |
| Non-DC xylitol | 26.6 | 28.6 | 38.6 | 48.6 | 53.6 |
| Purified water | 25 | 23 | 18 | 13 | 8 |
| Wheat fiber | 25 | 25 | 20 | 15 | 15 |
| Sodium carbonate | 5.0 | 5.0 | 5.0 | 5.0 | 5.0 |
| Flavor | 8.9 | 8.9 | 8.9 | 8.9 | 8.9 |
| High intensity sweetener | 1.0 | 1.0 | 1.0 | 1.0 | 1.0 |
| Potassium sorbate | 0.1 | 0.1 | 0.1 | 0.1 | 0.1 |
| Silicon dioxide | 2.0 | 2.0 | 2.0 | 2.0 | 2.0 |
| Total | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5C: Pouches

The pouch compositions are prepared from the ingredients in table 11 using preparation method described in example 3.

The pouch compositions are filled into pouches as described in example 4 (pouch material of examples 1A was used, but 1B could also have been applied).

**Table 11: Pouch compositions.**

| **Pouches** | **P20** | **P21** | **P22** | **P23** | **C6** |
|---|---|---|---|---|---|
| Amount of nicotine | 9.6 mg | 9.6 mg | 9.6 mg | 9.6 mg | 9.6 mg |
| Water content [wt%] | 27 | 25 | 20 | 15 | 10 |

| **Raw material** | **Content in weight percent** | | | | |
|---|---|---|---|---|---|
| Premix II | 14.6 | 14.6 | 14.6 | 14.6 | 14.6 |
| Non-DC xylitol | 21.4 | 23.4 | 33.4 | 43.4 | 48.4 |
| Purified water | 22 | 20 | 15 | 10 | 5 |
| Wheat fiber | 25 | 25 | 20 | 15 | 15 |
| Sodium carbonate | 5.0 | 5.0 | 5.0 | 5.0 | 5.0 |
| Flavor | 8.9 | 8.9 | 8.9 | 8.9 | 8.9 |
| High intensity sweetener | 1.0 | 1.0 | 1.0 | 1.0 | 1.0 |
| Potassium sorbate | 0.1 | 0.1 | 0.1 | 0.1 | 0.1 |
| Silicon dioxide | 2.0 | 2.0 | 2.0 | 2.0 | 2.0 |
| Total | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5D: Pouches

The pouch compositions are prepared from the ingredients in table 12 using preparation method described in example 3.

The pouch compositions are filled into pouches as described in example 4 (pouch material of examples 1A was used, but 1B could also have been applied).

**Table 12: Pouch compositions.**

| **Pouches** | **P30** | **P31** | **P32** | **P33** | **P34** | **P35** | **P36** | **P37** | **P38** |
|---|---|---|---|---|---|---|---|---|---|
| Amount of nicotine | 9.6 mg | 9.6 mg | 9.6 mg | 4.8 mg | 7.2 mg | 12.0 mg | 4.8 mg | 7.2 mg | 12.0 mg |
| Water content [wt%] | 27 | 27 | 27 | 27 | 27 | 27 | 27 | 27 | 27 |

| **Raw material** | **Content in weight percent** | | | | | | | | |
|---|---|---|---|---|---|---|---|---|---|
| NPR (16%) | 12.1 | - | - | 6.1 | 9.0 | 15.1 | - | - | - |
| Premix II | - | 14.6 | - | - | - | - | - | - | - |
| Premix VI | - | - | 6.4 | - | - | - | 3.2 | 4.8 | 8.0 |
| Non-DC xylitol | 18.9 | 21.4 | 26.6 | 24.9 | 22.0 | 15.9 | 28.6 | 27.5 | 25.2 |
| Purified water | 27 | 22 | 25 | 27 | 27 | 27 | 26.2 | 25.7 | 24.8 |
| Wheat fiber | 25 | 25 | 25 | 25 | 25 | 25 | 25 | 25 | 25 |
| Sodium carbonate | 5.0 | 5.0 | 5.0 | 5.0 | 5.0 | 5.0 | 5.0 | 5.0 | 5.0 |
| Flavor | 9.0 | 9.0 | 9.0 | 9.0 | 9.0 | 9.0 | 9.0 | 9.0 | 9.0 |
| High intensity sweetener | 1.0 | 1.0 | 1.0 | 1.0 | 1.0 | 1.0 | 1.0 | 1.0 | 1.0 |
| Silicon dioxide | 2.0 | 2.0 | 2.0 | 2.0 | 2.0 | 2.0 | 2.0 | 2.0 | 2.0 |
| Total | 100 | 100 | 100 | 100 | 100 | 100 | 100 | 100 | 100 |

Pouch content: 500 mg total.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Silicon dioxide is used as a glidant. Other possible glidants include e.g. magnesium stearate, starch and talc.

### Example 5E: Pouches

The pouch compositions are prepared from the ingredients in table 13 using preparation method described in example 3.

The pouch compositions are filled into pouches as described in example 4 (pouch material of examples 1A was used, but 1B could also have been applied).

**Table 13: Pouch compositions.**

| **Pouches** | **P40** | **P41** | **P42** | **P43** | **P44** | **P45** | **P46** |
|---|---|---|---|---|---|---|---|
| Amount of nicotine | 9.6 mg | 9.6 mg | 9.6 mg | 9.6 mg | 4.8 mg | 7.2 mg | 12.0 mg |
| Water content [wt%] | 27 | 27 | 27 | 27 | 27 | 27 | 27 |

| **Raw material** | **Content in weight percent** | | | | | | |
|---|---|---|---|---|---|---|---|
| NPR (16%) | - | 7.0 | - | - | - | - | - |
| NBT | 5.9 | 2.3 | 2.3 | 2.3 | - | - | - |
| Premix II | - | - | 8.5 | - | 7.3 | 10.9 | 18.2 |
| Premix VI | - | - | - | 3.7 | - | - | - |
| Non-DC xylitol | 25.1 | 21.7 | 23.1 | 26.0 | 26.2 | 23.8 | 19.0 |
| Purified water | 27 | 27 | 24.1 | 26.0 | 24.5 | 23.3 | 20.8 |
| Wheat fiber | 25 | 25 | 25 | 25 | 25 | 25 | 25 |
| Sodium carbonate | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 |
| High intensity sweetener | 1.0 | 1.0 | 1.0 | 1.0 | 1.0 | 1.0 | 1.0 |
| Potassium sorb ate | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 |
| Total | 100 | 100 | 100 | 100 | 100 | 100 | 100 |

Pouch content: 500 mg total.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5F: Pouches

The pouch compositions are prepared from the ingredients in table 14 using preparation method described in example 3.

The pouch compositions are filled into pouches as described in example 4 (pouch material of examples 1A was used, but 1B could also have been applied).

**Table 14: Pouch compositions.**

| **Pouches** | **P50** | **P51** | **P52** | **P53** | **P54** | **P55** | **P56** | **P57** | **P58** | **C14** |
|---|---|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg | 9.6 mg | 9.6 mg |
| Water content [wt%] | 30 | 30 | 30 | 30 | 30 | 30 | 30 | 30 | 30 | 35 |

| **Raw material** | **Content in weight percent** | | | | | | | | | |
|---|---|---|---|---|---|---|---|---|---|---|
| Premix VI | 6.4 | 6.4 | 6.4 | 6.4 | 6.4 | 6.4 | 6.4 | 6.4 | 6.4 | 6.4 |
| Non-DC xylitol | 5.0 | - | 5.0 | - | - | 7.0 | | 5.0 | 5.0 | - |
| Xylitol DC | - | - | - | - | - | - | 5.0 | - | - | - |
| Non-DC Isomalt | - | 21.6 | - | - | - | - | - | - | - | - |
| Isomalt DC | - | - | - | - | - | - | - | 16.6 | - | - |
| Sorbitol | - | - | 16.6 | - | - | - | - | - | - | - |
| Mannitol DC | - | - | - | - | - | - | - | - | 16.6 | - |
| Non-DC Mannitol | - | - | - | 21.6 | - | - | - | - | - | - |
| Non-DC Maltitol | - | - | - | - | 21.6 | - | - | - | - | - |
| Non-DC Erythritol | 16.6 | - | - | - | - | 14.6 | 16.6 | - | - | - |
| Purified water | 28 | 28 | 28 | 28 | 28 | 28 | 28 | 28 | 28 | 33 |
| Wheat fiber | 27 | 27 | 27 | 27 | 27 | 27 | 27 | 27 | 27 | 43.6 |
| Sodium carbonate | 5.0 | 5.0 | 5.0 | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 | 8.9 | 8.9 | 8.9 |
| High intensity sweetener | 1.0 | 1.0 | 1.0 | 1.0 | 1.0 | 1.0 | 1.0 | 1.0 | 1.0 | 1.0 |
| Potassium sorb ate | 0.1 | 0.1 | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 | 2.0 | 2.0 |
| Total | 100 | 100 | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5G: Pouches

The pouch compositions are prepared from the ingredients in table 15 using preparation method described in example 3.

The pouch compositions are filled into pouches as described in example 4 (pouch material of examples 1A was used, but 1B could also have been applied).

**Table 15: Pouch compositions.**

| **Pouches** | **P60** | **P61** | **P62** | **P63** | **P64** | **P65** | **P66** | **P67** | **P68** | **C15** |
|---|---|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg | 9.6 mg | 9.6 mg |
| Water content [wt%] | 27 | 27 | 27 | 27 | 27 | 27 | 27 | 27 | 27 | 35 |

| **Raw material** | **Content in weight percent** | | | | | | | | | |
|---|---|---|---|---|---|---|---|---|---|---|
| Premix II | 14.6 | 14.6 | 14.6 | 14.6 | 14.6 | 14.6 | 14.6 | 14.6 | 14.6 | 14.6 |
| Non-DC xylitol | 5.0 | - | 5.0 | - | - | 7.0 | | 5.0 | 5.0 | - |
| Xylitol DC | - | - | - | - | - | - | 5.0 | - | - | - |
| Non-DC Isomalt | - | 19.4 | - | - | - | - | - | - | - | - |
| Isomalt DC | - | - | - | - | - | - | - | 14.4 | - | - |
| Sorbitol | - | - | 14.4 | - | - | - | - | - | - | - |
| Mannitol DC | - | - | - | - | - | - | - | - | 14.4 | - |
| Non-DC Mannitol | - | - | - | 19.4 | - | - | - | - | - | - |
| Non-DC Maltitol | - | - | - | - | 19.4 | - | - | - | - | - |
| Non-DC Erythritol | 14.4 | - | - | - | - | 12.4 | 14.4 | - | - | - |
| Purified water | 22 | 22 | 22 | 22 | 22 | 22 | 22 | 22 | 22 | 33 |
| Wheat fiber | 27 | 27 | 27 | 27 | 27 | 27 | 27 | 27 | 27 | 35.4 |
| Sodium carbonate | 5.0 | 5.0 | 5.0 | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 | 8.9 | 8.9 | 8.9 |
| High intensity sweetener | 1.0 | 1.0 | 1.0 | 1.0 | 1.0 | 1.0 | 1.0 | 1.0 | 1.0 | 1.0 |
| Potassium sorb ate | 0.1 | 0.1 | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 | 2.0 | 2.0 |
| Total | 100 | 100 | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5H: Pouches

The pouch compositions are prepared from the ingredients in table 16 using preparation method described in example 3.

The pouch compositions are filled into pouches as described in example 4 (pouch material of examples 1A was used, but 1B could also have been applied).

**Table 16: Pouch compositions.**

| **Pouches** | **P70** | **P71** | **P72** | **P73** | **P74** | **P75** | **P76** | **P77** |
|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg |
| Water content [wt%] | 27 | 27 | 27 | 20 | 20 | 30 | 20 | 30 |

| **Raw material** | **Content in weight percent** | | | | | | | |
|---|---|---|---|---|---|---|---|---|
| Premix VI | 6.4 | 6.4 | 6.4 | 6.4 | 6.4 | 6.4 | 6.4 | 6.4 |
| Non-DC Xylitol | 26.5 | 26.5 | 26.5 | 50.5 | 30.5 | - | 30.5 | - |
| Xylitol DC | - | - | - | - | - | 5.0 | - | 5.0 |
| Non-DC Erythritol | - | - | - | - | - | 28.5 | - | 20.5 |
| Purified water | 25 | 25 | 25 | 18 | 18 | 28 | 18 | 28 |
| Wheat fiber | - | - | - | 10 | 30 | 15 | - | 15 |
| Oat fiber | 27 | - | - | - | - | - | 30 | - |
| Pea Fiber | - | 27 | - | - | - | - | - | - |
| Powdered Cellulose | - | - | 27 | - | - | - | - | - |
| Sodium carbonate | 5.0 | 5.0 | 5.0 | 5.0 | 5.0 | 3.5 | 5.0 | 5.0 |
| Sodium hydrogencarbonate | - | - | - | - | - | 3.5 | - | - |
| Flavor | 7.0 | 7.0 | 7.0 | 7.0 | 7.0 | 7.0 | 7.0 | 7.0 |
| NaCl | - | - | - | - | - | - | - | 10 |
| High intensity sweetener | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 |
| Total | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate and optionally sodium hydrogen carbonate are used as alkaline buffering agents. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 51: Pouches

The pouch compositions are prepared from the ingredients in table 17 using preparation method described in example 3.

The pouch compositions are filled into pouches as described in example 4 (pouch material of examples 1A was used, but 1B could also have been applied).

**Table 17: Pouch compositions.**

| **Pouches** | **P80** | **P81** | **P82** | **P83** | **P84** | **P85** | **P86** | **P87** |
|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg |
| Water content [wt%] | 27 | 27 | 27 | 20 | 20 | 35 | 20 | 35 |

| **Raw material** | **Content in weight percent** | | | | | | | |
|---|---|---|---|---|---|---|---|---|
| Premix II | 14.6 | 14.6 | 14.6 | 14.6 | 14.6 | 14.6 | 14.6 | 14.6 |
| Non-DC Xylitol | 21.3 | 21.3 | 21.3 | 45.3 | 25.3 | 20.3 | 25.3 | 20.3 |
| Purified water | 22 | 22 | 22 | 15 | 15 | 30 | 15 | 30 |
| Wheat fiber | - | - | - | 10 | 30 | 20 | - | - |
| Oat fiber | 27 | - | - | - | - | - | 30 | 20 |
| Pea Fiber | - | 27 | - | - | - | - | - | - |
| Powdered Cellulose | - | - | 27 | - | - | - | - | - |
| Sodium carbonate | 5.0 | 5.0 | 5.0 | 5.0 | 5.0 | 5.0 | 5.0 | 5.0 |
| Flavor | 7.0 | 7.0 | 7.0 | 7.0 | 7.0 | 7.0 | 7.0 | 7.0 |
| High intensity sweetener | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 |
| Total | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus" or "Vitacel 200WF".

Oat fiber, trade name "Vitacel HF 600".

Pea fiber, trade name "Vitacel EF150".

Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, powdered cellulose, cellulose fibers, apple fibers, cocoa fibers, bamboo fibers, bran fibers, and cellulose fiber. Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5J: Pouches

The pouch compositions are prepared from the ingredients in table 18 using preparation method described in example 3.

The pouch compositions are filled into pouches as described in example 4 (pouch material of examples 1A was used, but 1B could also have been applied).

**Table 18: Pouch compositions.**

| **Pouches** | **P90** | **P91** | **P92** | **P93** | **P94** | **P95** | **P96** | **P97** | **P98** |
|---|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg | 9.6 mg |
| Water content [wt%] | 27 | 27 | 27 | 27 | 27 | 27 | 27 | 27 | 27 |

| **Raw material** | **Content in weight percent** | | | | | | | | |
|---|---|---|---|---|---|---|---|---|---|
| NPR (16%) | 12.1 | 12.1 | 12.1 | 12.1 | 12.1 | 12.1 | 12.1 | 12.1 | 12.1 |
| Non-DC Xylitol | 5.0 | - | 5.0 | - | - | - | 5.0 | 5.0 | 5.0 |
| Xylitol DC | - | - | - | - | - | 5.0 | - | - | - |
| Non-DC Isomalt | - | 18.8 | - | - | - | - | - | - | - |
| Isomalt DC | - | - | - | - | - | - | - | - | - |
| Sorbitol | - | - | 13.8 | - | - | - | - | - | - |
| Non-DC Mannitol | - | - | - | 18.8 | - | - | - | - | - |
| Mannitol DC | - | - | - | - | - | - | - | - | - |
| Non-DC Maltitol | - | - | - | - | 18.8 | - | - | - | - |
| Non-DC Erythritol | 13.8 | - | - | - | - | 13.8 | 13.8 | 13.8 | 13.8 |
| Purified water | 27 | 27 | 27 | 27 | 27 | 27 | 27 | 27 | 27 |
| Wheat fiber | 27 | 27 | 27 | 27 | 27 | 27 | - | - | - |
| Oat fiber | - | - | - | - | - | - | 27 | - | - |
| Pea Fiber | - | - | - | - | - | - | - | 27 | - |
| Powdered Cellulose | - | - | - | - | - | - | - | - | 27 |
| Sodium carbonate | 5.0 | 5.0 | 5.0 | 5.0 | 5.0 | 5.0 | 5.0 | 5.0 | 5.0 |
| Flavor | 7.0 | 7.0 | 7.0 | 7.0 | 7.0 | 7.0 | 7.0 | 7.0 | 7.0 |
| High intensity sweetener | 1.0 | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 | 2.0 |
| Total | 100 | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus" or "Vitacel 200WF".

Oat fiber, trade name "Vitacel HF 600".

Powdered cellulose, trade name "Vitacel L00" or "Vitacel L700G".

Pea fiber, trade name "Vitacel EF150".

Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, powdered cellulose, cellulose fibers, apple fibers, cocoa fibers, bamboo fibers, bran fibers, and cellulose fiber. Sodium carbonate, sodium hydrogen carbonate and/or trometamol are used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5K: Pouches

The pouch compositions are prepared from the ingredients in table 19 using preparation method described in example 3.

The pouch compositions are filled into pouches as described in example 4 (pouch material of examples 1A was used, but 1B could also have been applied).

**Table 19: Pouch compositions.**

| **Pouches** | **P 100** | **P 101** | **P 102** | **P 103** | **P 104** | **P 105** | **P 106** | **P 107** | **C16** |
|---|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg | 9.6 mg |
| Water content [wt%] | 27 | 27 | 27 | 27 | 27 | 27 | 27 | 27 | 35 |

| **Raw material** | **Content in weight percent** | | | | | | | | |
|---|---|---|---|---|---|---|---|---|---|
| NPR (16%) | 12.1 | 12.1 | 12.1 | 12.1 | 12.1 | - | - | - | 12.1 |
| Premix VI | - | - | - | - | - | 6.4 | 6.4 | 6.4 | - |
| Non-DC Xylitol | 16.9 | 19.4 | 11.9 | 14.9 | 14.9 | 27.6 | 20.6 | 20.6 | - |
| Purified water | 27 | 27 | 27 | 27 | 27 | 27 | 27 | 27 | 35 |
| Wheat fiber | 27 | 27 | 27 | 27 | 27 | 27 | 27 | 27 | 37.8 |
| Sodium carbonate | 5.0 | 2.5 | 10.0 | 3.5 | - | - | 3.5 | - | 5.0 |
| Sodium hydrogencarbonat e | - | - | - | 3.5 | - | - | 3.5 | - | - |
| Trometamol | - | - | - | - | 7.0 | - | - | 7.0 | - |
| Flavor | 8.9 | 8.9 | 8.9 | 8.9 | 8.9 | 8.9 | 8.9 | 8.9 | 7.0 |
| High intensity sweetener | 1.0 | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 | 2.0 |
| Total | 100 | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate, sodium hydrogen carbonate and/or trometamol are used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5L: Pouches

The pouch compositions are prepared from the ingredients in table 20 using preparation method described in example 3.

The pouch compositions are filled into pouches as described in example 4 (pouch material of examples 1A was used, but 1B could also have been applied).

**Table 20: Pouch compositions.**

| **Pouches** | **P110** | **P111** | **P112** | **P113** | **P114** | **P115** | **P116** | **P117** |
|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg |
| Water content [wt%] | 30 | 30 | 30 | 30 | 30 | 30 | 30 | 27 |

| **Raw material** | **Content in weight percent** | | | | | | | |
|---|---|---|---|---|---|---|---|---|
| NPR (16%) | - | - | - | - | 12.1 | 12.1 | 12.1 | - |
| Premix II | 14.6 | 14.6 | - | - | - | - | - | 14.6 |
| Premix VI | - | - | 6.4 | 6.4 | - | - | - | - |
| CaCl₂ | 1.3 | 2.0 | 1.3 | 2.0 | 1.0 | 1.3 | 2.0 | - |
| Non-DC Xylitol | 12.1 | 11.4 | 17.3 | 16.6 | 9.9 | 9.6 | 9.9 | 7.0 |
| Non-DC Erythritol | - | - | - | - | - | - | - | 16.3 |
| Purified water | 25 | 25 | 28 | 28 | 30 | 30 | 30 | 22 |
| Wheat fiber | 30 | 30 | 30 | 30 | 30 | 30 | 30 | 27 |
| Sodium carbonate | 5.0 | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 | 5.0 |
| High intensity sweetener | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 |
| Total | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5M: Pouches

The pouch compositions are prepared from the ingredients in table 21 using preparation method described in example 3.

The pouch compositions are filled into pouches as described in example 4 (pouch material of examples 1A was used, but 1B could also have been applied).

**Table 21: Pouch compositions.**

| **Pouches** | **P120** | **P121** | **P122** | **P123** | **C17** | **C18** | **C19** | **C20** |
|---|---|---|---|---|---|---|---|---|
| Amount of nicotine | 9.6 mg | 9.6 mg | 9.6 mg | 9.6 mg | 9.6 mg | 9.6 mg | 9.6 mg | 9.6 mg |
| Water content [wt%] | 30 | 30 | 30 | 30 | 30 | 30 | 30 | 30 |

| **Raw material** | **Content in weight percent** | | | | | | | |
|---|---|---|---|---|---|---|---|---|
| Premix II | 14.6 | - | 14.6 | - | 14.6 | - | 14.6 | - |
| Premix VI | - | 6.4 | - | 6.4 | - | 6.4 | - | 6.4 |
| CaCl₂ | 1.0 | 1.0 | - | - | 1.0 | 1.0 | - | - |
| Non-DC Xylitol | 12.4 | 17.6 | 13.4 | 18.6 | 10.4 | 15.6 | 9.4 | 14.6 |
| Purified water | 25 | 28 | 25 | 28 | 25 | 28 | 25 | 28 |
| Wheat fiber | 30 | 30 | 30 | 30 | 30 | 30 | 30 | 30 |
| Sodium alginate | - | - | - | - | 2.0 | 2.0 | 2.0 | 2.0 |
| Sodium carbonate | 5.0 | 5.0 | 5.0 | 5.0 | 5.0 | 5.0 | 5.0 | 5.0 |
| Flavor | 8.9 | 8.9 | 8.9 | 8.9 | 8.9 | 8.9 | 8.9 | 8.9 |
| NaCl | - | - | - | - | - | - | 2.0 | 2.0 |
| High intensity sweetener | 1.0 | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 | 2.0 |
| Total | 100 | 100 | 100 | 100 | 100 | 100 | 100 | 100 |

Pouch content: 500 mg total, i.e. nicotine conc 19.2 mg/g.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, powdered cellulose, bran fibers, bamboo fibers, and cellulose fiber.

Sodium alginate, glycerol and hydroxypropyl cellulose (HPC) may be used as humectants. Other humectants as described herein may also be used in combination with sodium alginate, glycerol or HPC or as an alternative.

Sodium carbonate is used as an alkaline buffering agent. Other buffering agents as described herein may also be used in combination with sodium carbonate or an alternative.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 6A: Pouch release experiments (in vitro)

The release properties of the pouches were tested in an in vitro experiment. Reaction tubes having a diameter approx. 2 cm and containing 10 mL of 0.02 M potassium dihydrogen phosphate-buffer (pH adjusted to 7.4) were warmed to 37 degrees Celsius. One reaction tuber per timepoint was used.

A pouch was submerged in the buffer of the first reaction tube using tweezers. After a specified time period, the pouch was captured with the tweezer and gently swirled in the buffer before being removed from the first reaction tube and added to the next reaction tube, representing the next time point. The procedure was repeated until the desired number of time points had been tested.

The whole release experiment was performed at 37 degrees Celsius. No stirring or shaken was applied during the release experiment.

The amount of release nicotine was determined by analyzing the buffer samples at the different timepoints using standard HPLC.

### Example 6B: Release experiment on pouch P 120-123

The release experiment was performed as described in example 6A.

**Table 22: Shows the percentage of nicotine released from nicotine pouches at different timepoints with and without CaCl₂ and with or without alginate.**

| **Pouch** | **C19** | **C17** | **C20** | **C18** | **P122** | **P120** | **P123** | **P121** |
|---|---|---|---|---|---|---|---|---|
| **Nicotine premix** | II | II | VI | VI | II | II | VI | VI |
| **Humectant** | Algin ate | Algin ate | Algin ate | Algin ate | None | None | None | None |
| **CaCl₂ (wt%)** | - | 1.0 | - | 1.0 | - | 1.0 | - | 1.0 |

| **Min.** | **Released nicotine (%)** | | | | | | | |
|---|---|---|---|---|---|---|---|---|
| 2 | 13.8 | 12.9 | 20.8 | 24.0 | 16.2 | 29.5 | 26.9 | 38.7 |
| 5 | 25.7 | 26.0 | 39.8 | 42.9 | 28.6 | 50.9 | 47.4 | 58.2 |
| 10 | 37.5 | 40.3 | 59.0 | 61.7 | 42.0 | 64.8 | 66.0 | 74.8 |
| 30 | 60.4 | 62.3 | 79.4 | 82.6 | 59.2 | 78.7 | 79.5 | 92.2 |

Evaluation: the results demonstrate increased release of nicotine from pouches not comprising sodium alginate.

Also, the result shows that the presence of CaCl₂ increases the release of nicotine from pouches. The presence of CaCl₂ increases both the initial release rate and seems to also increase the effective release of nicotine.

Furthermore, the results demonstrate that increasing the nicotine content of the premixes also increases the nicotine release from the pouches.

### Example 7A: Weight-loss over time

Pouch compositions with different humectants (comparative formulations) and without humectants were prepared and directly placed in open containers at 25 degrees Celsius and 60% RH (relative humidity).

**Table 23: shows relative weight-loss in % from comparative pouch compositions with varying humectants and from pouch compositions without humectants.**

| | | **Time** | | | |
|---|---|---|---|---|---|
| **Pouch** | **Humectant** | **1 week** | **2 weeks** | **3 weeks** | **4 weeks** |
| P1 | - | 0.68 | 1.14 | 1.37 | 2.51 |
| C2 | Alginate | 0.96 | 1.53 | 2.11 | 3.64 |
| C3 | Glycerol | 1.05 | 1.68 | 2.10 | 3.77 |
| C4 | Hydroxypropyl cellulose | 1.15 | 2.08 | 2.54 | 4.39 |

Evaluation: the results demonstrate a higher weight-loss from pouch compositions comprising humectants selected from alginate, glycerol, and hydroxypropyl cellulose.

### Example 8A: User evaluation.

The produced pouches of the invention were evaluated and found highly suitable as delivery vehicles of nicotine in that they provide a favorable release of nicotine and at the same time are pleasant to the user, e.g. with respect to a desirable mouthfeel such as a moist and moldable texture and a desirable taste.

### Example 8B: User evaluation.

Pouches were evaluated by a test-panel of 4 trained assessors. Each assessor evaluated all samples twice. The test-panel evaluated the pouches on 2 different parameters over 15 min: overall taste and sweetness intensity.

The test was performed on pouches with two different flavors.

Pouch P117-1 corresponds to P117 with lemon and orange flavor.

Comparative P117-C1, which is based on P117, but with 2.0% alginate, 10.0% flavor (lemon and orange) and where the content of erythritol is adjusted to obtain a total of 100%.

Pouch P117-2 corresponds to P117 with raspberry and liquorice.

Comparative P117-C2, which is based on P117, but with 2.0% alginate, 10.0% flavor (raspberry and liquorice) and where the content of erythritol is adjusted to obtain a total of 100%.

**Table 24.**

| Minutes | Overall taste | | | | Sweetness intensity | | | |
|---|---|---|---|---|---|---|---|---|
| | P117-C1 | P117-1 | P117-C2 | P117-2 | P117-C1 | P117-1 | P117-C2 | P117-2 |
| 0.5 | 3.5 | 4 | 3 | 4 | 3.5 | 4 | 3 | 4 |
| 1 | 3.5 | 4 | 3 | 4 | 3.5 | 4 | 3 | 4 |
| 3 | 3.5 | 4.5 | 3.5 | 4.5 | 4 | 4.5 | 3 | 4.5 |
| 5 | 4 | 4 | 3.5 | 4 | 4 | 4 | 3 | 4 |
| 10 | 4 | 4 | 3.5 | 4 | 4 | 4 | 3 | 4 |
| 15 | 4 | 4 | 3.5 | 3.5 | 4 | 4 | 3 | 3.5 |

Evaluation: the test panel found that pouches P117-1 and P117-2 without alginate and a lower amount of flavor scored similarly or even higher on overall taste and sweetness intensity during the test period when compared to pouches P117-C1 and P117-C2 with alginate and a higher amount flavor.

Surprisingly, the inventive pouches P117-1 and P117-2 were found to score higher on both overall taste and sweetness during the initial time of use, i.e. use up to 5 min. Also, the inventive pouches P117-1 and P117-2 were found to score similarly or higher in the period from 5 to 15 min of use, despite the lower amount of flavor in the inventive pouches P117-1 and P117-2 tested.

## Claims

1. A non-tobacco oral nicotine pouch composition comprising
water in an amount of at least 15 % by weight of the pouch composition,
nicotine, and
at least one sugar alcohol,
wherein the pouch composition is free of humectants consisting of alginate, propylene glycol, hydroxypropyl cellulose and glycerol, and
wherein the at least one sugar alcohol comprises non-directly compressible (non-DC) grade sugar alcohol.

2. The non-tobacco oral nicotine pouch composition according to claim 1, wherein the pouch composition is further free of humectants consisting of modified starch, triacetin, polyethylene glycol (PEG), pectin, and xanthan gum.

3. The non-tobacco oral nicotine pouch composition according to claim 1 or 2, wherein the pouch composition comprises 0 - 0.4% by weight of humectants.

4. The non-tobacco oral nicotine pouch composition according to any of claims 1-3, wherein the pouch composition is free of humectants.

5. The non-tobacco oral nicotine pouch composition according to any of claims 1-4, wherein the pouch composition comprises the at least one sugar alcohol in an amount of at least 1% by weight of the composition, such as at least 2% by weight of the composition, such as at least 5% by weight of the composition, such as at least 10% by weight of the composition.

6. The non-tobacco oral nicotine pouch composition according to any of claims 1-5, wherein the at least one sugar alcohol comprises non-directly compressible (non-DC) grade sugar alcohol selected from the list consisting of xylitol, maltitol, mannitol, erythritol, isomalt, lactitol, and any combination thereof.

7. The non-tobacco oral nicotine pouch composition according to any of claims 1-6, wherein the non-directly compressible (non-DC) grade sugar alcohol(s) is provided as particles, which have not been subject to granulation or agglomeration steps.

8. The non-tobacco oral nicotine pouch composition according to any of claims 1-7, wherein the pouch composition comprises nicotine in an amount of at least 0.1% by weight, such as least 0.2% by weight of the pouch composition.

9. The non-tobacco oral nicotine pouch composition according to any of claims 1-8, wherein the pouch composition comprises nicotine selected from the group consisting of a nicotine salt, nicotine free base, nicotine-ion exchange resin combination, a nicotine inclusion complex or nicotine in any non-covalent binding; nicotine bound to zeolites; nicotine bound to cellulose, such as microcrystalline cellulose, or starch microspheres, and mixtures thereof.

10. The non-tobacco oral nicotine pouch composition according to any of claims 1-9, wherein the pouch composition comprises nicotine-ion exchange resin combination in an amount corresponding to 0.1 to 20% by weight of the pouch composition.

11. The non-tobacco oral nicotine pouch composition according to any of claims 9-10, wherein the nicotine-ion exchange resin combination comprises nicotine complexed with ion exchange resin.

12. The non-tobacco oral nicotine pouch composition according to any of claims 9-11, wherein the nicotine-ion exchange resin combination comprises free-base nicotine mixed with ion exchange resin.

13. The non-tobacco oral nicotine pouch composition according to any of claims 9-12, wherein the nicotine-ion exchange resin combination comprises nicotine in an amount of between 5 and 50% by weight.

14. The non-tobacco oral nicotine pouch composition according to any of claims 9-13, wherein the ion-exchange resin comprises one or more resin(s) selected from the group consisting of:
(i) a methacrylic, weakly acidic type of resin containing carboxylic functional groups,
(ii) a copolymer of methacrylic acid and divinylbenzene, said copolymer containing carboxylic functional groups,
(iii) a polystyrene, strongly acidic type of resin containing sulphonic functional groups,
(iv) a polystyrene, intermediate acidic type of resin containing phosphonic functional groups, and
(v) a combination thereof.

15. The non-tobacco oral nicotine pouch composition according to any of claims 1-14, wherein the ion exchange resin comprises polacrilex resin.

16. The non-tobacco oral nicotine pouch composition according to any of claims 1-15, wherein the pouch composition comprises nicotine salt.

17. The non-tobacco oral nicotine pouch composition according to any of claims 1-16, wherein the pouch composition comprises water in an amount of 15-65% by weight of the composition, such as 15-60% by weight of the composition, such as 15-50% by weight of the composition, such as 20-50% by weight of the composition, such as 20-40% by weight of the composition.

18. The non-tobacco oral nicotine pouch composition according to any of claims 1-17, wherein the pouch composition comprises at least one water-insoluble fiber.

19. The non-tobacco oral nicotine pouch composition according to any of claims 1-18, wherein the water-insoluble fiber is a non-tobacco fiber.

20. The non-tobacco oral nicotine pouch composition according to any of claims 1-19, wherein the water-insoluble fiber is a plant fiber.

21. The non-tobacco oral nicotine pouch composition according to any of claims 1-20, wherein the pouch composition comprises a pH regulating agent.

22. An oral pouched nicotine product comprising a saliva-permeable pouch and the pouch composition of any of claims 1 - 21 enclosed in said pouch.

## Patentansprüche

1. Tabakfreie orale Nikotinbeutelzusammensetzung, umfassend
Wasser in einer Menge von wenigstens 15 Gew.-% der Beutelzusammensetzung,
Nikotin und
wenigstens einen Zuckeralkohol,
wobei die Beutelzusammensetzung frei von Feuchthaltemitteln ist, die aus Alginat, Propylenglycol, Hydroxypropylcellulose und Glycerin bestehen, und
wobei der wenigstens eine Zuckeralkohol nicht direkt komprimierbaren (nicht-DC-)Zuckeralkohol umfasst.

2. Tabakfreie orale Nikotinbeutelzusammensetzung nach Anspruch 1, wobei die Beutelzusammensetzung ferner frei von Feuchthaltemitteln ist, die aus modifizierter Stärke, Triacetin, Polyethylenglykol (PEG), Pektin und Xanthangummi bestehen.

3. Tabakfreie orale Nikotinbeutelzusammensetzung nach Anspruch 1 oder 2, wobei die Beutelzusammensetzung 0-0,4 Gew.-% Feuchthaltemittel umfasst.

4. Tabakfreie orale Nikotinbeutelzusammensetzung nach einem der Ansprüche 1-3, wobei die Beutelzusammensetzung frei von Feuchthaltemitteln ist.

5. Tabakfreie orale Nikotinbeutelzusammensetzung nach einem der Ansprüche 1-4, wobei die Beutelzusammensetzung den wenigstens einen Zuckeralkohol in einer Menge von wenigstens 1 Gew.-% der Zusammensetzung, wie wenigstens 2 Gew.-% der Zusammensetzung, wie wenigstens 5 Gew.-% der Zusammensetzung, wie wenigstens 10 Gew.-% der Zusammensetzung, umfasst.

6. Tabakfreie orale Nikotinbeutelzusammensetzung nach einem der Ansprüche 1-5, wobei der wenigstens eine Zuckeralkohol einen nicht direkt komprimierbaren (nicht-DC-)Zuckeralkohol umfasst, der ausgewählt ist aus der Liste bestehend aus Xylit, Maltit, Mannit, Erythrit, Isomalt, Lactit und einer beliebigen Kombination davon.

7. Tabakfreie orale Nikotinbeutelzusammensetzung nach einem der Ansprüche 1-6, wobei der/die nicht direkt komprimierbare(n) (nicht-DC-)Zuckeralkohol(e) als Partikel vorgesehen ist/sind, die keinen Granulations- oder Agglomerationsschritten unterworfen wurden.

8. Tabakfreie orale Nikotinbeutelzusammensetzung nach einem der Ansprüche 1-7, wobei die Beutelzusammensetzung Nikotin in einer Menge von wenigstens 0,1 Gew.%, wie wenigstens 0,2 Gew.-% der Beutelzusammensetzung aufweist.

9. Tabakfreie orale Nikotinbeutelzusammensetzung nach einem der Ansprüche 1-8, wobei die Beutelzusammensetzung Nikotin aufweist, ausgewählt aus der Gruppe bestehend aus einem Nikotinsalz, einer nikotinfreien Base, einer Nikotin-Ionenaustauschharz-Kombination, einem Nikotineinschlusskomplex oder Nikotin in einer nichtkovalenten Bindung; an Zeolithe gebundenem Nikotin; an Zellulose gebundenem Nikotin, wie mikrokristalliner Zellulose oder Stärkemikrosphären, und Mischungen davon.

10. Tabakfreie orale Nikotinbeutelzusammensetzung nach einem der Ansprüche 1-9, wobei die Beutelzusammensetzung eine Nikotin-Ionen-Austauschkombination in einer Menge aufweist, die 0,1 bis 20 Gew.- % der Beutelzusammensetzung entspricht.

11. Tabakfreie orale Nikotinbeutelzusammensetzung nach einem der Ansprüche 9-10, wobei die Nikotin-Ionenaustauschharz-Kombination Nikotin aufweist, das mit Ionenaustauschharz komplexiert ist.

12. Tabakfreie orale Nikotinbeutel-Zusammensetzung nach einem der Ansprüche 9-11, wobei die Nikotin-Ionenaustauschharz-Kombination Nikotin auf freier Basis, gemischt mit Ionenaustauschharz, aufweist.

13. Tabakfreie orale Nikotinbeutelzusammensetzung nach einem der Ansprüche 9-12, wobei die Nikotin-Ionenaustauschharz-Kombination Nikotin in einer Menge zwischen 5 und 50 Gew.-% aufweist.

14. Tabakfreie orale Nikotinbeutelzusammensetzung nach einem der Ansprüche 9-13, wobei das Ionenaustauschharz ein oder mehrere Harz(e) umfasst, ausgewählt aus der Gruppe bestehend aus:
(i) einem Methacrylharz vom schwach sauren Typ, das funktionelle Carboxylgruppen enthält,
(ii) einem Copolymer aus Methacrylsäure und Divinylbenzol, wobei das Copolymer funktionelle Carboxylgruppen enthält,
(iii) einem Polystyrolharz vom stark sauren Typ, das funktionelle Sulfongruppen enthält,
(iv) einem Polystyrolharz vom intermediären sauren Typ, das funktionelle Phosphongruppen enthält, und
(v) einer Kombination davon.

15. Tabakfreie orale Nikotinbeutelzusammensetzung nach einem der Ansprüche 1-14, wobei das Ionenaustauschharz ein Polacrilexharz aufweist.

16. Tabakfreie orale Nikotinbeutelzusammensetzung nach einem der Ansprüche 1-15, wobei die Beutelzusammensetzung Nikotinsalz aufweist.

17. Tabakfreie orale Nikotinbeutelzusammensetzung nach einem der Ansprüche 1-16, wobei die Beutelzusammensetzung Wasser in einer Menge von 15-65 Gew.-% der Zusammensetzung, wie 15-60 Gew.-% der Zusammensetzung, wie 15-50 Gew.-% der Zusammensetzung, wie 20-50 Gew.-% der Zusammensetzung, wie 20-40 Gew.-% der Zusammensetzung aufweist.

18. Tabakfreie orale Nikotinbeutelzusammensetzung nach einem der Ansprüche 1-17, wobei die Beutelzusammensetzung wenigstens eine wasserunlösliche Faser aufweist.

19. Tabakfreie orale Nikotinbeutelzusammensetzung nach einem der Ansprüche 1-18, wobei die wasserunlösliche Faser eine tabakfreie Faser ist.

20. Tabakfreie orale Nikotinbeutelzusammensetzung nach einem der Ansprüche 1-19, wobei die wasserunlösliche Faser eine Pflanzenfaser ist.

21. Tabakfreie orale Nikotinbeutelzusammensetzung nach einem der Ansprüche 1-20, wobei die Beutelzusammensetzung ein pH-Regulierungsmittel aufweist.

22. Orales Nikotinprodukt in einem Beutel, umfassend einen speicheldurchlässigen Beutel und die in diesem Beutel eingeschlossene Beutelzusammensetzung nach einem der Ansprüche 1-21.

## Revendications

1. Composition en sachet de nicotine orale sans tabac comprenant
de l'eau en une quantité d'au moins 15 % en poids de la composition en sachet,
de la nicotine, et
au moins un alcool de sucre,
dans laquelle la composition en sachet est exempte d'humectants constitués d'alginate, de propylène glycol, d'hydroxypropylcellulose et de glycérol, et
dans laquelle l'au moins un alcool de sucre comprend un alcool de sucre de qualité non compressible directement (non DC).

2. Composition en sachet de nicotine orale sans tabac selon la revendication 1, dans laquelle la composition en sachet est en outre exempte d'humectants constitués d'amidon modifié, de triacétine, de polyéthylène glycol (PEG), de pectine et de gomme de xanthane.

3. Composition en sachet de nicotine orale sans tabac selon la revendication 1 ou 2, dans laquelle la composition en sachet comprend 0 à 0,4 % en poids d'humectants.

4. Composition en sachet de nicotine orale sans tabac selon l'une quelconque des revendications 1 à 3, dans laquelle la composition en sachet est exempte d'humectants.

5. Composition en sachet de nicotine orale sans tabac selon l'une quelconque des revendications 1 à 4, dans laquelle la composition en sachet comprend l'au moins un alcool de sucre en une quantité d'au moins 1 % en poids de la composition, telle qu'au moins 2 % en poids de la composition, telle qu'au moins 5 % en poids de la composition, telle qu'au moins 10 % en poids de la composition.

6. Composition en sachet de nicotine orale sans tabac selon l'une quelconque des revendications 1 à 5, dans laquelle l'au moins un alcool de sucre comprend un alcool de sucre de qualité non compressible directement (non DC) choisi dans la liste constituée par le xylitol, le maltitol, le mannitol, l'érythritol, l'isomalt, le lactitol et toute combinaison de ceux-ci.

7. Composition en sachet de nicotine orale sans tabac selon l'une quelconque des revendications 1 à 6, dans laquelle le ou les alcool(s) de sucre de qualité non compressible directement (non DC) est/sont fourni(s) sous forme de particules, qui n'ont pas été soumises à des étapes de granulation ou d'agglomération.

8. Composition en sachet de nicotine orale sans tabac selon l'une quelconque des revendications 1 à 7, dans laquelle la composition en sachet comprend de la nicotine en une quantité d'au moins 0,1 % en poids, telle qu'au moins 0,2 % en poids de la composition en sachet.

9. Composition en sachet de nicotine orale sans tabac selon l'une quelconque des revendications 1 à 8, dans laquelle la composition en sachet comprend de la nicotine choisie dans le groupe constitué par un sel de nicotine, une base libre de nicotine, une combinaison de nicotine-résine échangeuse d'ions, un complexe d'inclusion de nicotine ou de la nicotine dans une liaison non covalente quelconque ; de la nicotine liée à des zéolithes ; de la nicotine liée à de la cellulose, telle que de la cellulose microcristalline, ou des microsphères d'amidon ou des mélanges de ceux-ci.

10. Composition en sachet de nicotine orale sans tabac selon l'une quelconque des revendications 1 à 9, dans laquelle la composition en sachet comprend une combinaison de nicotine-résine échangeuse d'ions en une quantité correspondant à 0,1 à 20 % en poids de la composition en sachet.

11. Composition en sachet de nicotine orale sans tabac selon l'une quelconque des revendications 9 et 10, dans laquelle la combinaison de nicotine-résine échangeuse d'ion comprend de la nicotine complexée avec une résine échangeuse d'ions.

12. Composition en sachet de nicotine orale sans tabac selon l'une quelconque des revendications 9 à 11, dans laquelle la combinaison de nicotine-résine échangeuse d'ion comprend de la nicotine en base libre mélangée avec une résine échangeuse d'ions.

13. Composition en sachet de nicotine orale sans tabac selon l'une quelconque des revendications 9 à 12, dans laquelle la combinaison de nicotine-résine échangeuse d'ions comprend de la nicotine en une quantité d'entre 5 et 50 % en poids.

14. Composition en sachet de nicotine orale sans tabac selon l'une quelconque des revendications 9 à 13, dans laquelle la résine échangeuse d'ions comprend une ou plusieurs résines choisies dans le groupe constitué de :
(i) une résine méthacrylique de type acide faible contenant des groupes fonctionnels carboxyliques,
(ii) un copolymère d'acide méthacrylique et de divinylbenzène, ledit copolymère contenant des groupes fonctionnels carboxyliques,
(iii) une résine de polystyrène de type acide fort contenant des groupes fonctionnels sulfoniques,
(iv) une résine de polystyrène de type acide intermédiaire de résine contenant des groupes fonctionnels phosphoniques, et
(v) une combinaison de ceux-ci.

15. Composition en sachet de nicotine orale sans tabac selon l'une quelconque des revendications 1 à 14, dans laquelle la résine échangeuse d'ions comprend de la résine de polacrilex.

16. Composition en sachet de nicotine orale sans tabac selon l'une quelconque des revendications 1 à 15, dans laquelle la composition en sachet comprend un sel de nicotine.

17. Composition en sachet de nicotine orale sans tabac selon l'une quelconque des revendications 1 à 16, dans laquelle la composition en sachet comprend de l'eau en une quantité de 15 à 65 % en poids de la composition, telle que 15 à 60 % en poids de la composition, telle que 15 à 50 % en poids de la composition, telle que 20 à 50 % en poids de la composition, telle que 20 à 40 % en poids de la composition.

18. Composition en sachet de nicotine orale en sachet selon l'une quelconque des revendications 1 à 17, dans laquelle la composition en sachet comprend au moins une fibre insoluble dans l'eau.

19. Composition en sachet de nicotine orale sans tabac selon l'une quelconque des revendications 1 à 18, dans laquelle la fibre insoluble dans l'eau est une fibre sans tabac.

20. Composition en sachet de nicotine orale sans tabac selon l'une quelconque des revendications 1 à 19, dans laquelle la fibre insoluble dans l'eau est une fibre végétale.

21. Composition en sachet de nicotine orale sans tabac selon l'une quelconque des revendications 1 à 20, dans laquelle la composition en sachet comprend un agent de régulation du pH.

22. Produit en sachet à base de nicotine orale comprenant un sachet perméable à la salive et la composition en sachet selon l'une quelconque des revendications 1 à 21 enfermée dans ledit sachet.
